# EUROPÄISCHE PATENTSCHRIFT

(11) **EP 4 113 643 B1**
(45) Veröffentlichungstag und Bekanntmachung des Hinweises auf die Patenterteilung: **09.08.2023**
(21) Anmeldenummer: 22190898.1
(22) Anmeldetag: 06.10.2017
(51) Int. Cl.: H10K 85/60, H10K 50/18

(54) **SPIRO[FLUOREN-9,9'-(THIO)XANTHEN] VERBINDUNGEN**
SPIRO[FLUORENE-9,9'-(THIO)XANTHENE] COMPOUNDS
COMPOSÉS SPIRO[FLUORÈNE-9,9'-(THIO)XANTHÈNE]

(30) Priorität: 10.10.2016 EP 16193116
(43) Veröffentlichungstag der Anmeldung: 04.01.2023
(62) Teilanmeldung aus: 17787893.1
(73) Patentinhaber: Merck Patent GmbH, 64293 Darmstadt (DE)
(72) Erfinder: Voges, Frank, 64293 Darmstadt (DE); Mujica-Fernaud, Elvira, 64293 Darmstadt (DE); Montenegro, Elvira, 64293 Darmstadt (DE); Eberle, Thomas, 64293 Darmstadt (DE)

(56) Entgegenhaltungen:
- WO-A1-2016/062368
- CN-A- 105 924 395
- KR-A- 20150 106 501

## Beschreibung

Die vorliegende Anmeldung betrifft eine Xanthen- oder Thioxanthen-Verbindung einer weiter unten definierten Formel. Weiterhin betrifft die Anmeldung eine elektronische Vorrichtung, welche eine oben genannte Verbindung enthält.

Die elektronische Vorrichtung ist bevorzugt eine organische Elektrolumineszenzvorrichtung (OLED). Weiterhin betrifft die Anmeldung die Verwendung in den oben genannten Vorrichtungen.

Unter elektronischen Vorrichtungen im Sinne dieser Anmeldung werden organische elektronische Vorrichtungen verstanden (organic electronic devices), d.h. Vorrichtungen, die organische Halbleitermaterialien als Funktionsmaterialien enthalten. Insbesondere werden darunter OLEDs verstanden. Unter der Bezeichnung OLEDs werden elektronische Vorrichtungen verstanden, welche eine oder mehrere Schichten enthaltend organische Verbindungen aufweisen und unter Anlegen von elektrischer Spannung Licht emittieren. Der Aufbau und das allgemeine Funktionsprinzip von OLEDs sind dem Fachmann bekannt.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Schichten mit lochtransportierender Funktion. Zu diesen Schichten zählen unter anderem lochinjizierende Schichten, Lochtransportschichten und Elektronenblockierschichten. Zur Verwendung in diesen Schichten werden weiterhin neue Materialien mit lochtransportierenden Eigenschaften gesucht.

Zusätzlich besteht Bedarf an neuen Device-Aufbauten, sowie an neuen Kombinationen von Funktionsmaterialien in verschiedenen Schichten der OLEDs. Dabei sind insbesondere die Schichten mit lochtransportierender Funktion, ihre Zusammensetzung und ihre Abfolge von Bedeutung, um die Leistungsdaten von OLEDs zu verbessern.

Im Stand der Technik, beispielsweise in den Offenlegungsschriften WO 2014/072017 A1, KR 2015 0106501 A, CN 105 924 395 A, WO 2016/062368 A1 und CN 103 666 454 A, sind Xanthen- und ThioxanthenVerbindungen, die eine Arylaminogruppe tragen, als OLED-Funktionsmaterialien beschrieben.

Gegenüber den dort beschriebenen OLED-Aufbauten, welche die genannten Verbindungen enthalten, besteht jedoch noch Verbesserungsbedarf bezüglich der Leistungsdaten der OLEDs, insbesondere Betriebsspannung, Lebensdauer und Effizienz.

Weiterhin besteht noch Verbesserungsbedarf bezüglich der dort offenbarten konkreten Verbindungen.

Im Rahmen der vorliegenden Erfindung wurde gefunden, dass OLEDs, welche bestimmte Xanthen- oder Thioxanthen-Verbindungen in einer an die Anode angrenzenden Schicht enthalten, oder diese Verbindungen in einer Schicht enthalten, welche mindestens zwei weitere Schichten zwischen dieser Schicht und der anodennächsten emittierenden Schicht aufweist, hervorragende Leistungsdaten aufweisen.

Weiterhin wurde gefunden, dass bestimmte neue Xanthen- oder Thioxanthen-Verbindungen hervorragende Leistungsdaten aufweisen.

Gegenstand der vorliegenden Erfindung ist daher eine Verbindung einer Formel (I) 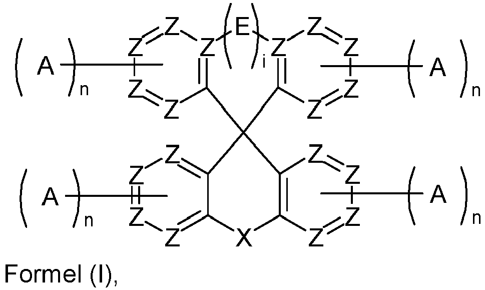 wobei gilt:
- A: entspricht einer Formel (A) gemäß Anspruch 1;
- E: ist eine Einfachbindung;
- X: ist O oder S;
- Z: ist bei jedem Auftreten gleich oder verschieden CR² oder N oder C, wobei eine Gruppe Z genau dann gleich C ist, wenn eine Gruppe A oder E an die betreffende Gruppe Z gebunden ist;
- R¹: ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können;
- R²: ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können;
- R³: ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
- R⁴: ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
- i: ist gleich 1
- n: ist bei jedem Auftreten gleich oder verschieden 0 oder 1, wobei die Summe aller Indices n gleich 1, 2, 3 oder 4 ist.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyloder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einem aromatischen Ringsystem mit 6 bis 40 aromatischen Ringatomen oder einem heteroaromatischen Ringsystem mit 5 bis 40 aromatischen Ringatomen, werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

In der Verbindung der Formel (I) ist X bevorzugt gleich O.

Weiterhin ist die Summe der Indices n in Formel (I) bevorzugt gleich 1 oder 2, besonders bevorzugt gleich 1.

Weiterhin sind bevorzugt höchstens 2 Gruppen Z pro Ring gleich N.

Weiterhin bevorzugt sind höchstens 4 Gruppen Z pro Verbindung der Formel (I), ganz besonders bevorzugt höchstens 2 Gruppen Z pro Verbindung der Formel (I) gleich Z.

Besonders bevorzugt ist Z gleich CR², wobei in dem Fall, dass eine Gruppe A oder E an die betreffende Gruppe Z gebunden ist, diese Gruppe Z gleich C ist.

Bevorzugt ist R¹ bei jedem Auftreten gleich oder verschieden gewählt aus H, D, F, CN, Si(R³)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R³ substituiert sein können; und wobei in den genannten Alkyl- oder Alkoxygruppen eine oder mehrere CH₂-Gruppen durch -C=C-, -R³C=CR³-, Si(R³)₂, C=O, C=NR³, -NR³-, -O-, -S-, -C(=O)O- oder -C(=O)NR³- ersetzt sein können.

Besonders bevorzugt ist R¹ bei jedem Auftreten gleich oder verschieden gewählt aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R³ substituiert sein können.

Bevorzugt ist R² bei jedem Auftreten gleich oder verschieden gewählt aus H, D, F, CN, Si(R³)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R³ substituiert sein können; und wobei in den genannten Alkyl- oder Alkoxygruppen eine oder mehrere CH₂-Gruppen durch -C=C-, -R³C=CR³-, Si(R³)₂, C=O, C=NR³, -NR³-, -O-, -S-, -C(=O)O- oder -C(=O)NR³- ersetzt sein können.

Besonders bevorzugt ist R² bei jedem Auftreten gleich oder verschieden gewählt aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R³ substituiert sein können.

Ganz besonders bevorzugt ist R² gleich H.

Bevorzugt ist R³ bei jedem Auftreten gleich oder verschieden gewählt aus H, D, F, CN, Si(R⁴)₃, geradkettigen Alkyl- oder Alkoxygruppen mit 1 bis 20 C-Atomen, verzweigten oder cyclischen Alkyl- oder Alkoxygruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl- und Alkoxygruppen, die genannten aromatischen Ringsysteme und die genannten heteroaromatischen Ringsysteme jeweils mit einem oder mehreren Resten R⁴ substituiert sein können; und wobei in den genannten Alkyl- oder Alkoxygruppen eine oder mehrere CH₂-Gruppen durch -C≡C-, -R⁴C=CR⁴-, Si(R⁴)₂, C=O, C=NR⁴, -NR⁴-, -O-, -S-, -C(=O)O- oder -C(=O)NR⁴- ersetzt sein können.

Besonders bevorzugt ist R³ bei jedem Auftreten gleich oder verschieden gewählt aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁴ substituiert sein können.

Die Gruppe A entspricht einer Formel (A) 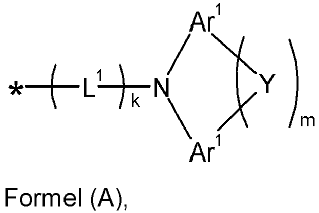 wobei gilt:
- Ar¹: ist Dibenzofuranyl, das mit einem oder mehreren Resten R¹ substituiert sein kann;
- Y: ist gewählt aus einer Einfachbindung, BR¹, C(R¹)₂, C(R¹)₂-C(R¹)₂, Si(R¹)₂, Si(R¹)₂-Si(R¹)₂, C=O, C=NR¹, C=C(R¹)₂, C(=O)N(R¹), O, S, S=O, SO₂ und NR¹;
- k: ist gleich 0;
- m: ist gleich 0 oder 1;
wobei die Gruppe A über die mit * markierte Bindung an den Rest der Verbindung der Formel (I) gebunden ist.

Weiterhin bevorzugt ist in Formel (A) m gleich 0, d.h. die beiden Gruppen Ar¹ sind nicht miteinander verbunden.

Bevorzugte Gruppen Ar¹ sind in der folgenden Tabelle abgebildet:

| | | |
|---|---|---|
| | 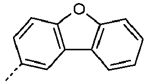 | 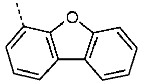 |
| | Ar¹-59 | Ar¹-60 |
| 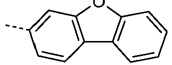 | 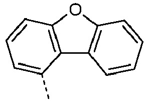 | |
| Ar¹-61 | Ar¹-62 | |

Die oben gezeigten Gruppen können an ihren unsubstituiert gezeichneten Positionen jeweils mit Resten R¹ substituiert sein.

Weiterhin ist in Formel (A) die Gruppe Y bevorzugt gewählt aus einer Einfachbindung, C(R¹)₂, O, S und NR¹. Besonders bevorzugt ist Y eine Einfachbindung.

Eine bevorzugte Ausführungsform der Verbindung der Formel (I) entspricht der folgenden Formel (I-1) 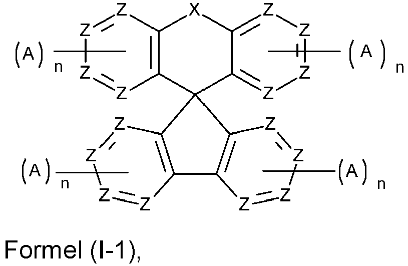 wobei die auftretenden Variablen definiert sind wie oben. Bevorzugt entsprechen die auftretenden Variablen ihren oben genannten bevorzugten Ausführungsformen.

Besonders bevorzugte Ausführungsformen der Verbindungen der Formel (I) entsprechen den folgenden Formeln 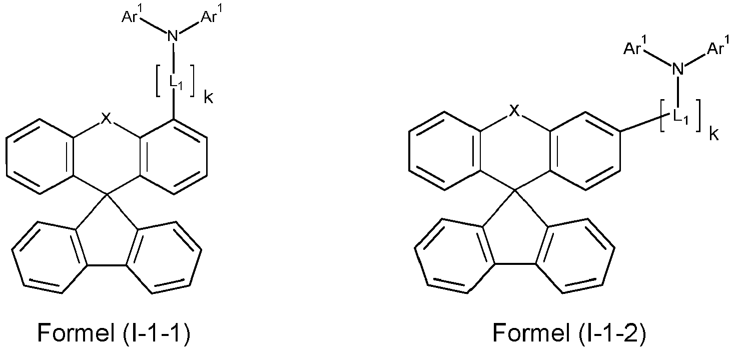 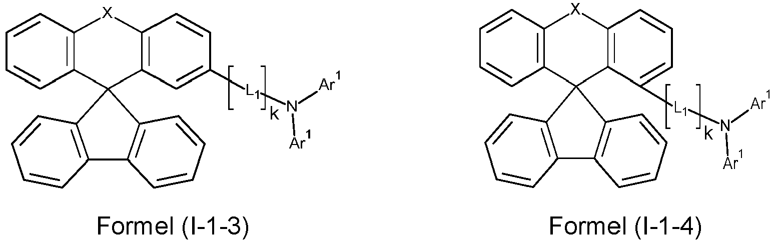 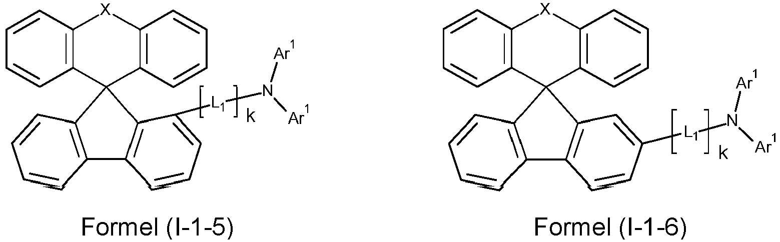 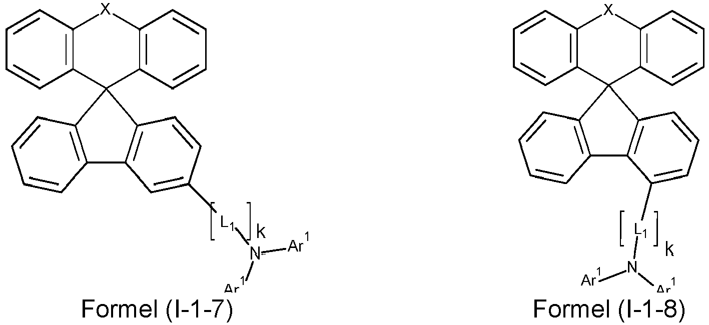 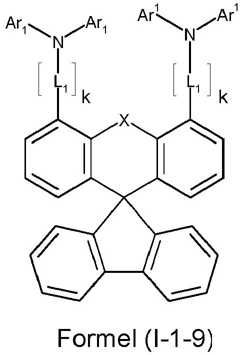 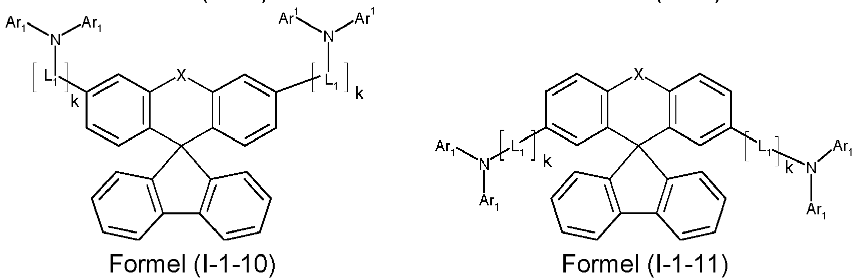 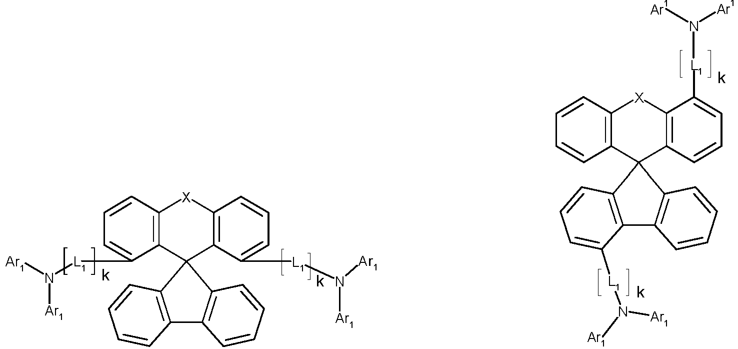 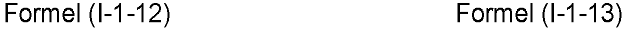 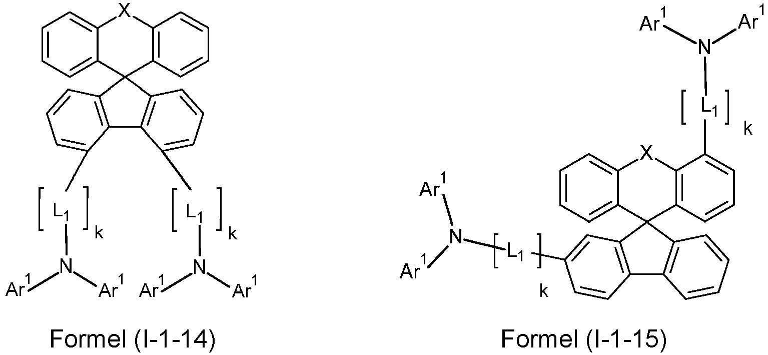 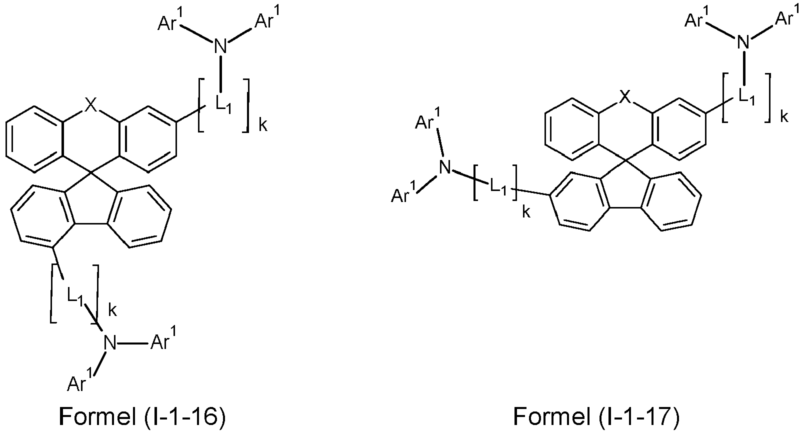 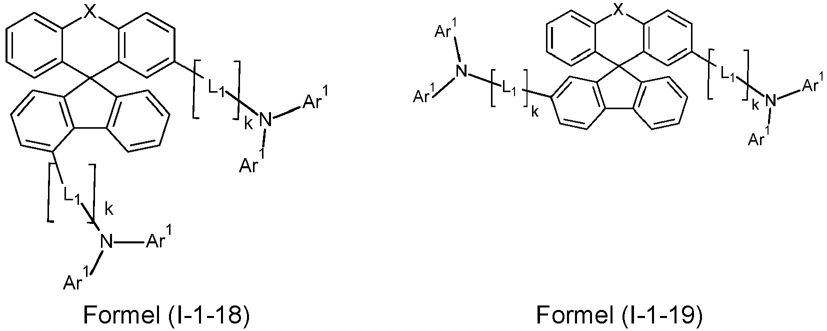 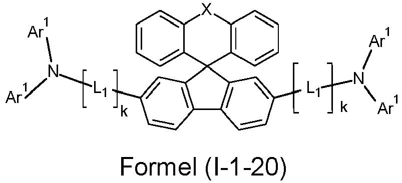 wobei die auftretenden Variablen wie obenstehend definiert sind, und wobei die Verbindungen an den unsubstituiert gezeichneten Positionen an den Benzolringen jeweils mit Resten R² substituiert sein können. Bevorzugt sind die Verbindungen an den unsubstituiert gezeichneten Positionen an den Benzolringen jeweils unsubstituiert.

Ganz besonders bevorzugt entspricht die Verbindung einer der Formeln (I-1-1) bis (I-1-8), am stärksten bevorzugt einer der Formeln (I-1-1) bis (I-1-3). Für derartige Verbindungen wurden besonders gute Leistungsdaten bei Verwendung in der erfindungsgemäßen Vorrichtung festgestellt.

Besonders bevorzugt ist die Kombination der Formeln (1-1-1) bis (1-1-20) mit den bevorzugten Ausführungsformen von Ar¹.

Bevorzugte Ausführungsformen von Verbindungen der Formel (I) sind die Folgenden:

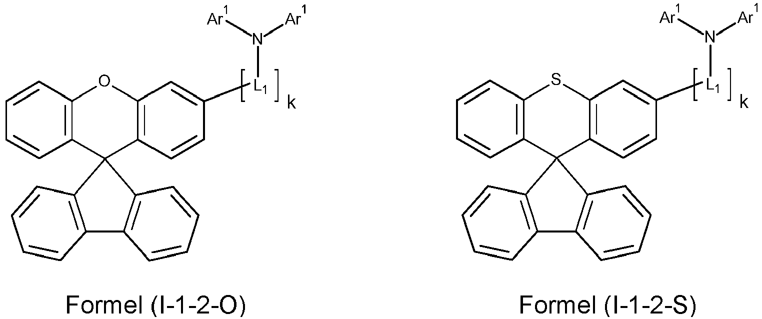

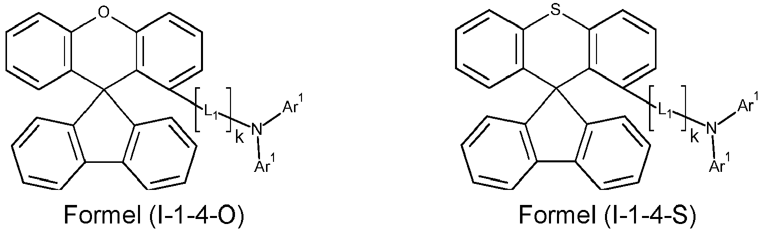

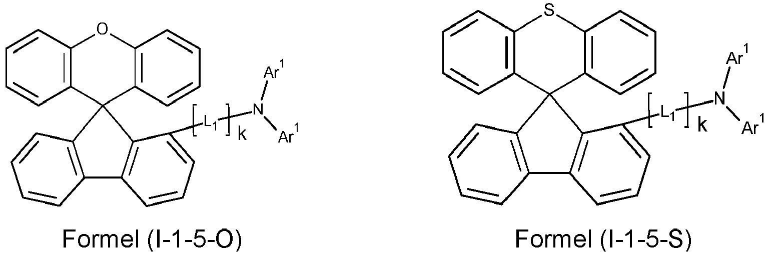

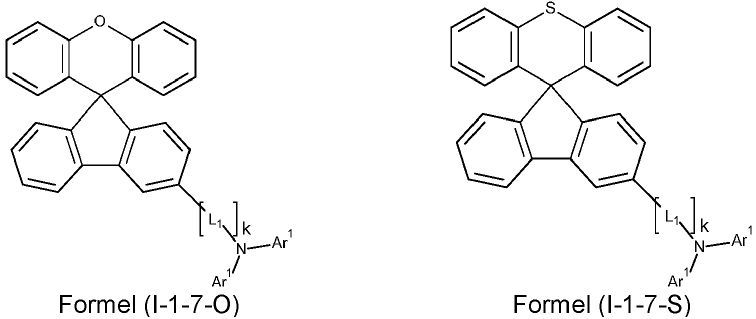

Für die Synthese der Verbindungen der Formel (I) können im Stand der Technik bekannte Verfahren genutzt werden, insbesondere Verfahren, die in der Offenlegungsschrift WO 2014/072017 offenbart sind.

Die erfindungsgemäße Vorrichtung ist bevorzugt gewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs). Besonders bevorzugt ist sie eine organische Elektrolumineszenzvorrichtung.

In der erfindungsgemäßen elektronischen Vorrichtung ist die Verbindung der Formel (I) bevorzugt in einer Schicht enthalten, die an die Anode angrenzend angeordnet ist. Diese Schicht enthält bevorzugt einen p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 und DE 102012209523 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden. Bevorzugt liegen p-Dotanden in einem Anteil von insgesamt 0.5% bis 10 Vol.-%, bevorzugt 0.8 bis 8 % Vol.-% in der betreffenden Schicht vor.

Bevorzugt sind als p-Dotanden insbesondere die folgenden Verbindungen:

| | | |
|---|---|---|
| 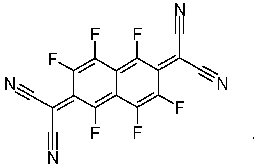 | 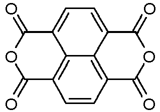 | 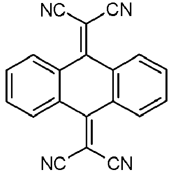 |
| (D-1) | (D-2) | (D-3) |
| 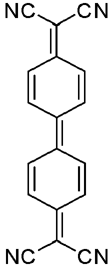 | 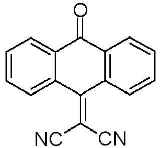 | 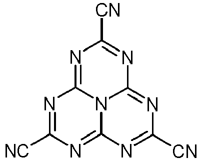 |
| (D-4) | (D-5) | (D-6) |
| 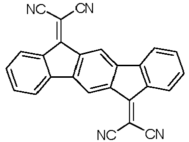 | 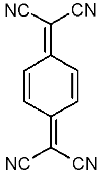 | 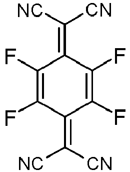 |
| (D-7) | (D-8) | (D-9) |
| 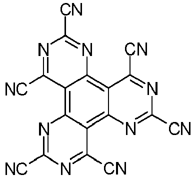 | 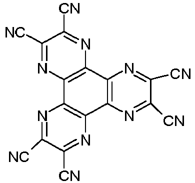 | 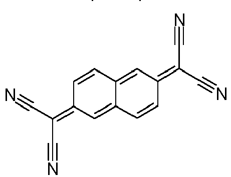 |
| (D-10) | (D-11) | (D-12) |
| 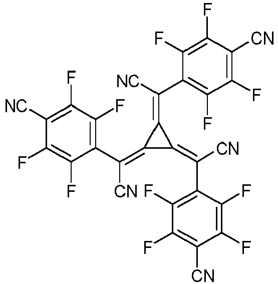 | | |
| (D-13) | | |

Bevorzugt ist eine elektronische Vorrichtung, welche in der genannten Reihenfolge die folgenden Schichten enthält: Anode, lochtransportierende Schicht HTL1, lochtransportierende Schicht HTL2, lochtransportierende Schicht HTL3, emittierende Schicht EML, elektronentransportierende Schicht ETL, und Kathode, wobei weitere Schichten vorhanden sein können, wobei die Schicht HTL1 an die Anode angrenzt, wobei die Schicht HTL3 an die emittierende Schicht angrenzt, und wobei die Schicht HTL1 eine Verbindung der Formel (I) enthält. Bevorzugt enthält dabei die Schicht HTL3 keine Verbindung der Formel (I).

Eine besonders bevorzugte Ausführungsform der Vorrichtung weist die folgende Schichtabfolge zwischen Anode und anodennächster emittierender Schicht auf: Anode, lochtransportierende Schicht HTL1 enthaltend eine Verbindung der Formel (I), lochtransportierende Schicht HTL2, lochtransportierende Schicht HTL3 enthaltend keine Verbindung der Formel (I), anodennächste emittierende Schicht. Dabei sind bevorzugt keine weiteren Schichten zwischen Anode und anodennächster emittierender Schicht vorhanden. Die Schicht HTL1 weist dabei bevorzugt eine Dicke von 5 bis 50 nm auf. Die Schicht HTL2 weist dabei bevorzugt eine Dicke von 5 bis 250 nm auf. Die Schicht HTL3 weist dabei bevorzugt eine Dicke von 5 bis 120 nm auf.

Eine alternative besonders bevorzugte Ausführungsform der Vorrichtung weist die folgende Schichtabfolge zwischen Anode und anodennächster emittierender Schicht auf: Anode, lochtransportierende Schicht HTL1 enthaltend eine Verbindung der Formel (I) und einen p-Dotanden, lochtransportierende Schicht HTL2, lochtransportierende Schicht HTL3 enthaltend keine Verbindung der Formel (I), anodennächste emittierende Schicht. Dabei sind bevorzugt keine weiteren Schichten zwischen Anode und anodennächster emittierender Schicht vorhanden. Die Schicht HTL1 weist dabei bevorzugt eine Dicke von 5 bis 250 nm auf. Die Schicht HTL2 weist dabei bevorzugt eine Dicke von 5 bis 250 nm auf. Die Schicht HTL3 weist dabei bevorzugt eine Dicke von 5 bis 120 nm auf.

Eine alternative besonders bevorzugte Ausführungform der Vorrichtung weist die folgende Schichtabfolge zwischen Anode und anodennächster emittierender Schicht auf: Anode, lochtransportierende Schicht HTL1 enthaltend eine Verbindung der Formel (I) und einen p-Dotanden, lochtransportierende Schicht HTL2a, lochtransportierende Schicht HTL2b enthaltend einen p-Dotanden, lochtransportierende Schicht HTL3 enthaltend keine Verbindung der Formel (I), anodennächste emittierende Schicht. Dabei sind bevorzugt keine weiteren Schichten zwischen Anode und anodennächster emittierender Schicht vorhanden.

Die lochtransportierende Schicht, die anodenseitig an die anodennächste emittierende Schicht angrenzt, enthält bevorzugt eine Monoamin-Verbindung. Unter einer Monoamin-Verbindung wird dabei eine Verbindung verstanden, die nur eine Aminogruppe enthält. Bevorzugt ist diese Aminogruppe eine Diarylaminogruppe. Unter einer Diarylaminogruppe wird eine Gruppe verstanden, in der an das Amino-Stickstoffatom zwei Gruppen gewählt aus Arylgruppen und Heteroarylgruppen gebunden sind.

Besonders bevorzugt enthält die lochtransportierende Schicht, die anodenseitig an die anodennächste emittierende Schicht angrenzt, eine Monoamin-Verbindung, die mindestens eine Gruppe gewählt aus Spirobifluorenylgruppen, Phenanthrenylgruppen, Fluorenylgruppen, Carbazolylgruppen, Dibenzofuranylgruppen und Dibenzothiophenylgruppen enthält. Besonders bevorzugt sind darunter Spirobifluorenyl-Monoamine, welche an einer der Positionen 1, 3 und 4 am Spirobifluoren-Grundgerüst eine Diarylaminogruppe tragen, darunter insbesondere die Verbindungen, die in der Offenlegungsschrift WO 2013/120577 auf den Seiten 36-51 und 88-122 offenbart sind. Ganz besonders bevorzugt sind Spirobifluorenyl-Monoamine, die an Position 4 am Spirobifluoren-Grundgerüst eine Diarylaminogruppe tragen, darunter insbesondere die Verbindungen, die in der Offenlegungsschrift WO 2013/120577 auf den Seiten 36-51 und 88-122 offenbart sind.

Es ist bevorzugt, dass die Monoamin-Verbindung, die in der lochtransportierenden Schicht enthalten ist, welche anodenseitig an die anodennächste emittierende Schicht angrenzt, ein HOMO-Energieniveau von 5.0 bis 5.6 eV aufweist, besonders bevorzugt 5.1 bis 5.5 eV aufweist. Das HOMO-Energieniveau wird dabei mittels Cyclovoltammetrie (CV) bestimmt, nach dem auf S. 28, Z.1 bis S. 29, Z. 21 der Offenlegungsschrift WO 2011/032624 beschriebenen Verfahren.

Die Vorrichtung kann zusätzlich zu den genannten Schichten weitere Schichten aufweisen, darunter insbesondere Schichten gewählt aus Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) und organischen oder anorganischen p/n-Übergängen.

Die Vorrichtung enthält bevorzugt nur eine emittierende Schicht. Sie kann jedoch auch mehrere emittierende Schichten enthalten. In diesem Fall weisen diese mehreren emittierenden Schichten bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen.

Die emittierende Schicht der Vorrichtung kann eine fluoreszierende emittierende Schicht sein, oder sie kann eine phosphoreszierende emittierende Schicht sein.

Unter phosphoreszierenden emittierenden Schichten werden insbesondere Schichten verstanden, welche mindestens einen phosphoreszierenden Emitter enthalten. Vom Begriff phosphoreszierende Emitter sind Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Emitter (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Emitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platinoder Kupferkomplexe als phosphoreszierende Emitter angesehen.

Bevorzugt ist die phosphoreszierende emittierende Schicht der Vorrichtung eine grün oder rot phosphoreszierende Schicht. Weiterhin bevorzugt ist die fluoreszierende emittierende Schicht der Vorrichtung eine blau fluoreszierende Schicht.

Die emittierenden Schichten enthalten bevorzugt mindestens ein Matrixmaterial und mindestens einen Emitter.

Insbesondere im Fall von phosphoreszierenden emittierenden Schichten ist es bevorzugt, dass die betreffende Schicht zwei oder mehr unterschiedliche Matrixmaterialien enthält, bevorzugt zwei oder drei, ganz besonders bevorzugt zwei (Mixed-Matrix-Systeme). Bevorzugt stellt dabei eines der beiden Matrixmaterialien ein Material mit lochtransportierenden Eigenschaften und das andere Matrixmaterial ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen.

Im Folgenden ist offenbart, welche Materialklassen bevorzugt in den betreffenden Funktionsschichten der Vorrichtung verwendet werden.

Bevorzugte phosphoreszierende Emitter zur Verwendung in der emittierenden Schicht können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind.

Bevorzugte fluoreszierende Emitter sind ausgewählt aus der Klasse der Arylamine. Unter einem Arylamin bzw. einem aromatischen Amin im Sinne dieser Erfindung wird dabei eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine, die in WO 2014/111269 und in WO 2017/036574 offenbarten erweiterten Benzoindenofluorene, die in WO 2017/028940 und WO 2017/028941 offenbarten Phenoxazine, und die in WO 2016/150544 offenbarten Fluoren-Derivate, die mit Furan-Einheiten oder mit Thiophen-Einheiten verbunden sind.

Als Matrixmaterialien, bevorzugt für fluoreszierende emittierende Schichten, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, die in EP 1749809, EP 1905754 und US 2012/0187826 offenbarten Pyren-Verbindungen, die in WO 2015/158409 offenbarten Benzanthracenyl-Anthracen-Verbindungen, die in WO 2017/025165 offenbarten Indeno-Benzofurane, und die in WO 2017/036573 offenbarten Phenanthryl-Anthracene.

Bevorzugte Matrixmaterialien für phosphoreszierende emittierende Verbindungen sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Weitere Verbindungen, die neben den Verbindungen der Formel (I) bevorzugt in lochtransportierenden Schichten der erfindungsgemäßen OLEDs eingesetzt werden, sind insbesondere Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder WO 2013/120577), Fluoren-Amine (z. B. gemäß WO 2014/015937, WO 2014/015938, WO 2014/015935 und WO 2015/082056), Spiro-Dibenzopyran-Amine (z. B. gemäß WO 2013/083216), Dihydroacridin-Derivate (z. B. gemäß WO 2012/150001), Spirodibenzofurane und Spirodibenzothiophene, z.B. gemäß WO 2015/022051 und WO 2016/102048 und WO 2016/131521, Phenanthren-Diarylamine, z.B. gemäß WO 2015/131976, Spiro-Tribenzotropolone, z.B. gemäß WO 2016/087017, Spirobifluorene mit meta-Phenyldiamingruppen, z.B. gemäß WO 2016/078738, Spiro-Bisacridine, zB. gemäß WO 2015/158411, Xanthen-Diarylamine, z.B. gemäß WO 2014/072017, und 9,10-Dihydroanthracen-Spiroverbindungen mit Diarylaminogruppen gemäß WO 2015/086108.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen in Displays, als Lichtquellen in Beleuchtungsanwendungen sowie als Lichtquellen in medizinischen und/oder kosmetischen Anwendungen (z.B. Lichttherapie) eingesetzt werden.

Die Verbindungen der Formel (I) können mittels üblicher Synthesemethoden der organischen Chemie hergestellt werden. Insbesondere werden dabei Buchwald- und Suzuki-Reaktionen, nukleophile Additionsreaktionen an Carbonylgruppen sowie Ringschlussreaktionen durch elektrophile aromatische Substitution eingesetzt.

Ein bevorzugtes Verfahren zur Herstellung von Verbindungen der Formel (I) verläuft wie folgt: Zunächst wird eine metallierte Ether- oder Thioether-Verbindung (B in untenstehendem Schema 1) an ein Keton C addiert, gefolgt von einer Ringschlussreaktion. Anschließend wird eine Aminogruppe oder eine Arylgruppe, die eine Aminogruppe enthält, über eine Buchwald- bzw. eine Suzuki-Reaktion eingefügt. Die metallierte Ether- bzw. Thioether-Verbindung ist bevorzugt eine lithiierte Verbindung oder eine entsprechende Grignard-Verbindung.

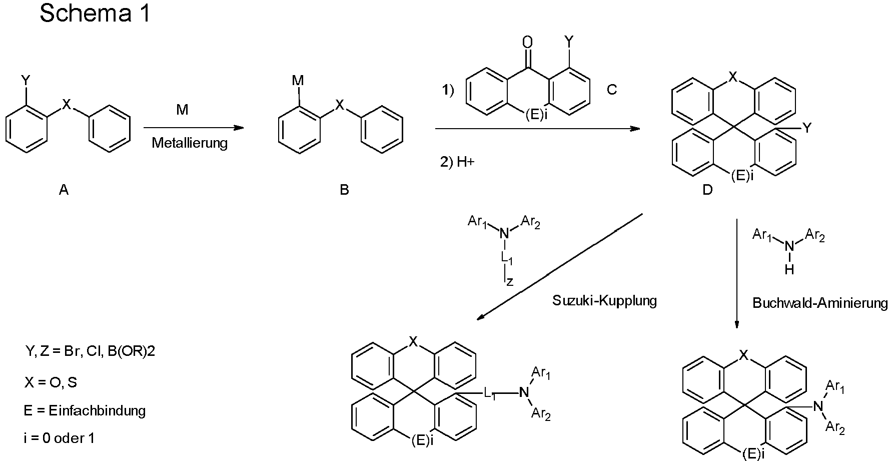

Alternativ können die Addition der metallierten Ether- bzw. Thioether-Gruppe an das Keton und die Ringschluss-Reaktion auch im Anschluss an eine Suzuki- oder Buchwald-Kupplung am Keton stattfinden, wie in Schema 2 gezeigt ist.

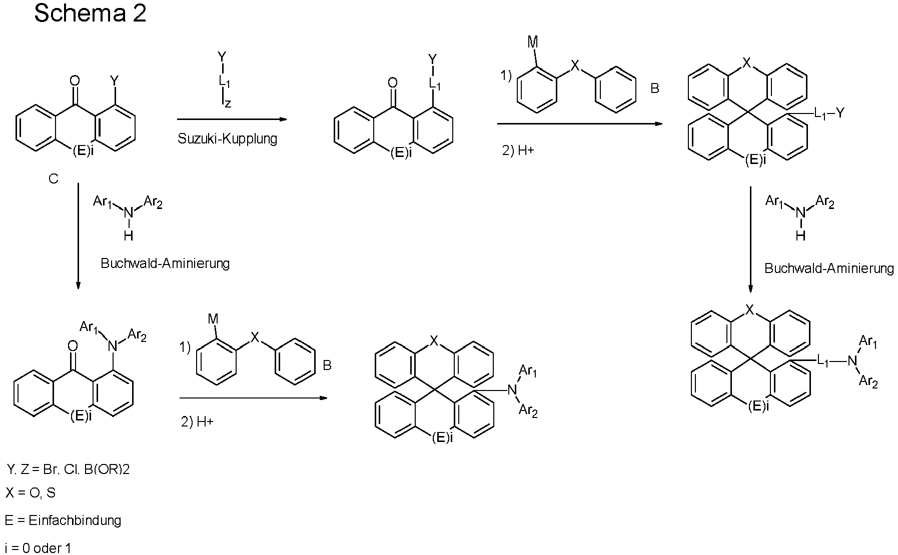

Gegenstand der Anmeldung ist damit ein Verfahren zur Herstellung einer Verbindung der Formel (I), dadurch gekennzeichnet, dass es eine Addition einer metallierten Ether- oder Thioether-Verbindung an ein Diarylketon und eine anschließende Ringschlussreaktion umfasst. Die metallierte Ether- oder Thioether-Verbindung ist bevorzugt eine metallierte Diarylether- oder Diarylthioether-Verbindung, ganz besonders bevorzugt eine lithiierte Diarylether- oder Diarylthioether-Verbindung oder ein entsprechendes Grignard-Derivat der Diarylether- oder Diarylthioether-Verbindung.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Anmeldung sind daher Oligomere, Polymere oder Dendrimere, enthaltend eine oder mehrere Verbindungen gemäß Formel (I), wobei die Bindung(en) zum Polymer, Oligomer oder Dendrimer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die Suzuki-Polymerisation, die Yamamoto-Polymerisation; die Stille-Polymerisation; und die Hartwig-Buchwald-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, a-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel

Gegenstand der Anmeldung ist daher weiterhin eine Formulierung, insbesondere eine Lösung, Dispersion oder Emulsion, enthaltend mindestens eine Verbindung gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Zusätzlich sind die Verbindungen der Formel (I) auch besonders geeignet, um in einer Elektronenblockierschicht einer OLED eingesetzt zu werden.

### Ausführungsbeispiele

### A) Synthesebeispiele

### Beispiel 1-1:

### Synthese der erfindungsgemäßen Verbindung 1-1 und Varianten (nicht Bestandteil der beanspruchten Erfindung)

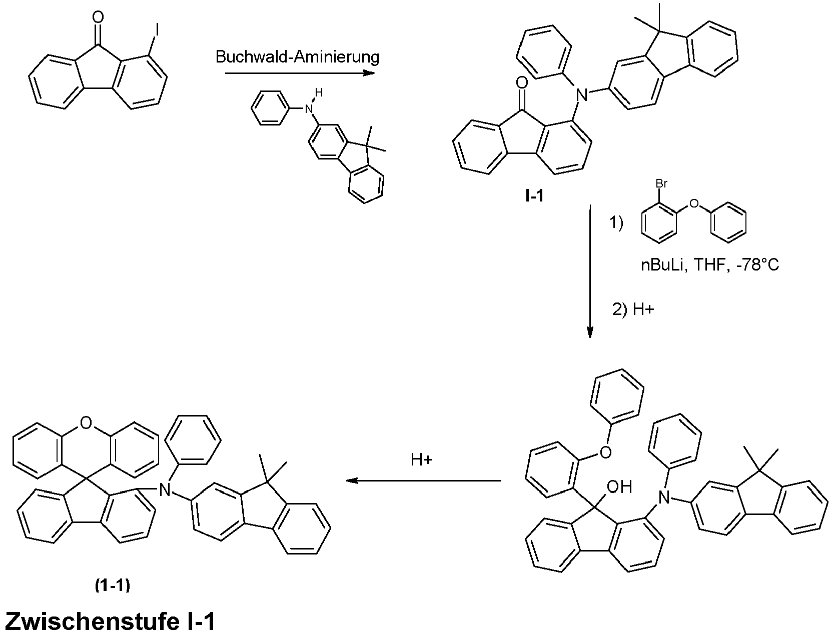

26,8g Phenyl-(9,9-dimethyl-9H-fluoren-2-yl)-amin (87,6 mmol) und 25 g lod-Benzofluorenon (87,6 mmol) werden in 700 mL Toluol gelöst. Die Lösung wird entgast und mit N₂ gesättigt. Danach wird sie mit 3,5 mL (3,5 mmol) einer Tri-tert-Butylphosphin 1M-Lösung und 0,46 g (1,75 mmol) Palladium(II)acetat versetzt und anschließend werden 16,8 g Natrium-tert-butylat (175 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 33 g (81% d. Th).

Analog dazu werden folgende Verbindungen hergestellt:
(nicht Bestandteil der beanspruchten Erfindung)

| | Edukt 1 | Edukt 2 | Produkt | Ausbeute |
|---|---|---|---|---|
| **I-2** | 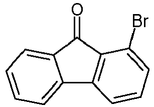 | 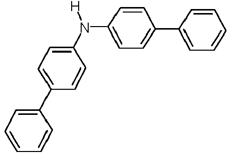 | 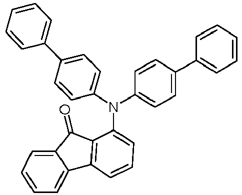 | 85% |
| **I-3** | 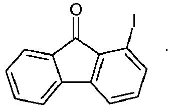 | 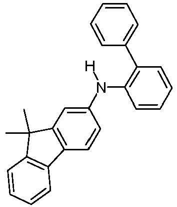 | 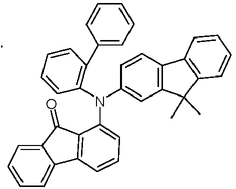 | 71% |
| **I-4** | 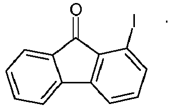 | 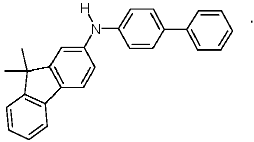 | 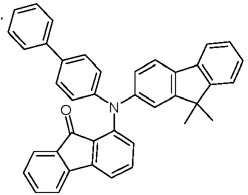 | 82% |
| **I-5** | 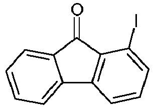 | 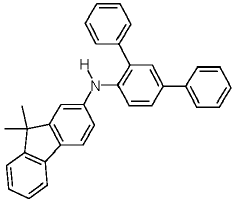 | 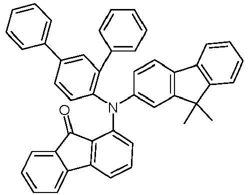 | 72% |
| **I-6** | 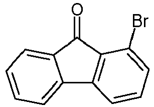 | 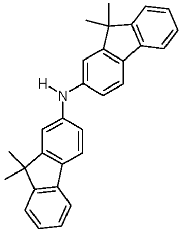 | 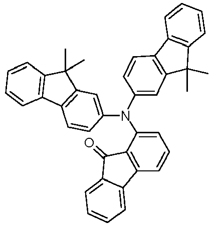 | 74% |
| **I-7** | 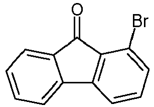 | 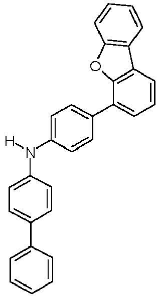 | 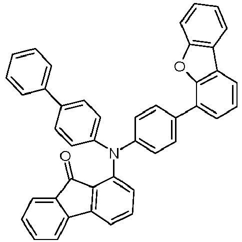 | 74% |
| **I-8** | 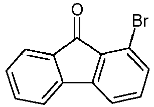 | 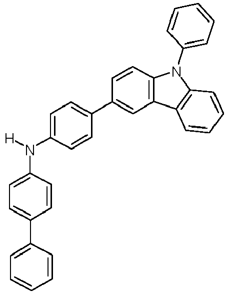 | 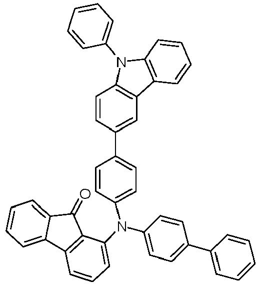 | 62% |
| **I-9** | 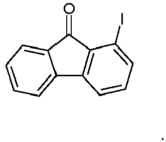 | 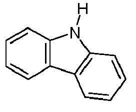 | 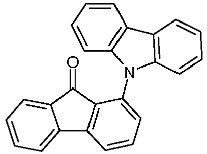 | 35% |
| **I-10** | 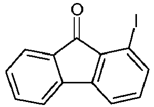 | 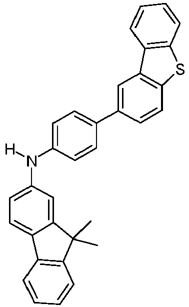 | 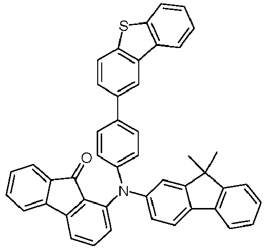 | 70% |
| **I-11** | 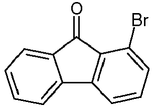 | 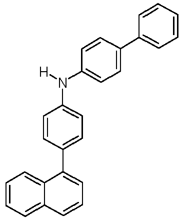 | 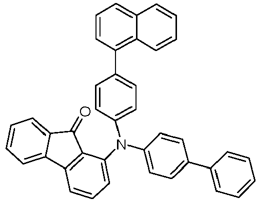 | 67% |

### Verbindung 1-1 (nicht Bestandteil der beanspruchten Erfindung)

17,37 g (69,6 mmol) 1-Bromo-2-diphenylether werden in einem ausgeheizten Kolben in 300 mL getrocknetem THF gelöst. Die Reaktionsmischung wird auf -78°C gekühlt. Bei dieser Temperatur werden 30 mL einer 2,5M-Lösung n-BuLi in Hexan (69,7 mmol) langsam zugetropft. Der Ansatz wird 1 Stunde bei -70°C nachgerührt. Anschließend werden 30 g des Bromfluorenon Derivates (63 mmol) in 200 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt.

Die einrotierte Lösung wird vorsichtig mit 300 ml Essigsäure versetzt und anschließend werden 20 ml rauchende HCl zugegeben. Der Ansatz wird auf 75°C erhitzt und 6 Stunden dort gehalten. Dabei fällt ein weißer Feststoff aus. Der Ansatz wird nun auf Raumtemperatur abgekühlt und der ausgefallene Feststoff wird abgesaugt und mit Wasser und Methanol nachgewaschen. Ausbeute: 35 g (88%)

Der Feststoff wird aus Heptan/Toluol umkristallisiert und abschließend im Hochvakuum sublimiert.

Analog dazu werden folgende Verbindungen hergestellt:
(nicht Bestandteil der beanspruchten Erfindung)

| | Edukt 1 | Edukt 2 | Produkt | Ausbeute |
|---|---|---|---|---|
| **1-2** | 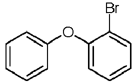 | 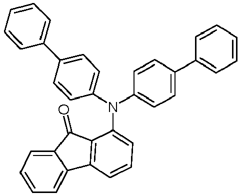 | 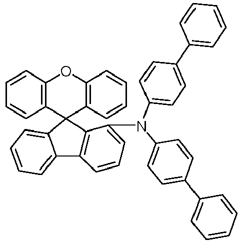 | 70% |
| **1-3** | 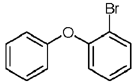 | 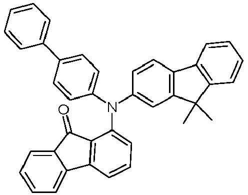 | 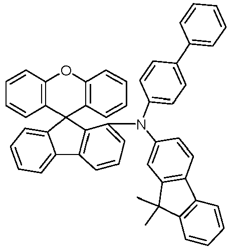 | 77% |
| **1-5** | 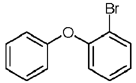 | 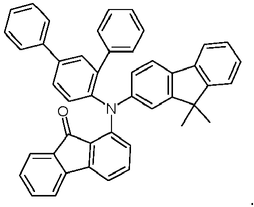 | 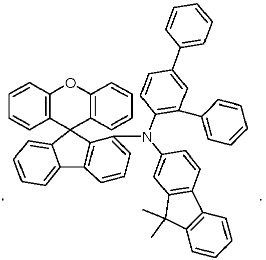 | 65% |
| **1-6** | 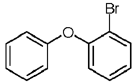 | 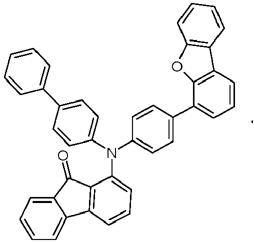 | 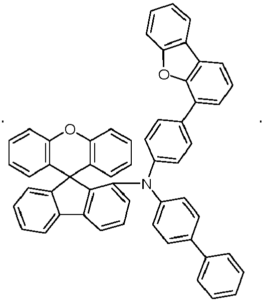 | 69% |
| **1-7** | 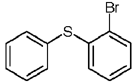 | 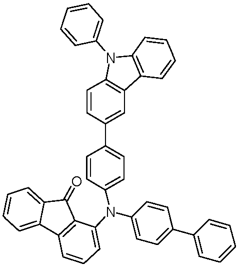 | 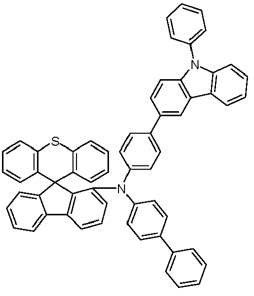 | 79% |
| **1-8** | 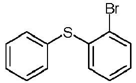 | 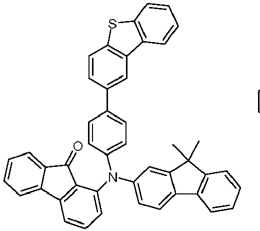 | 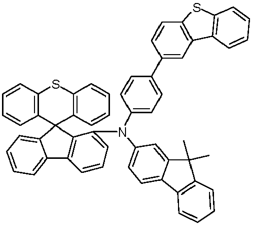 | 81% |
| **1-9** | 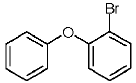 | 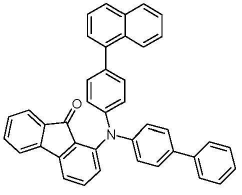 | 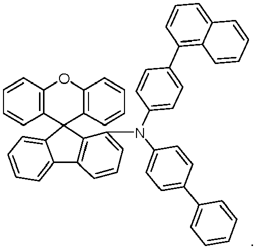 | 80% |
| **1-10** | 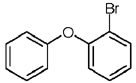 | 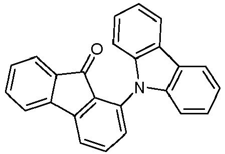 | 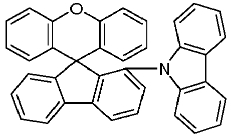 | 40% |
| **1-11** | 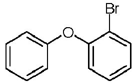 | 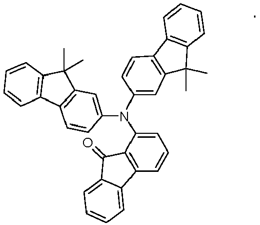 | 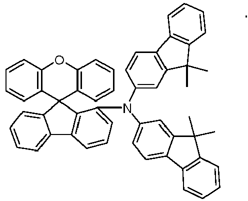 | 79% |

### Beispiel 2-1:

### Synthese der erfindungsgemäßen Verbindung 2-1 und Varianten (nicht Bestandteil der beanspruchten Erfindung)

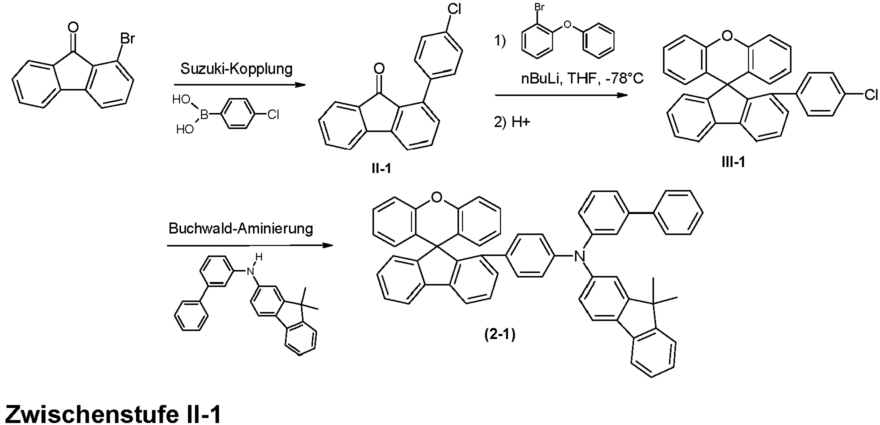

38 g 4-Chlorophenylboronsäure (243 mmol) und 60 g 1-Brom-fluoren-9-on (232 mmol) werden in 800 mL THF suspendiert. 230 ml von 2 M Kaliumcarbonat Lösung werden langsam zugetropft. Die Lösung wird entgast und mit N₂ gesättigt. Danach wird sie mit 8 g (7 mmol) Pd(Ph3P)4 versetzt. Die Reaktionsmischung wird 16 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus MeOH umkristallisiert. Die Ausbeute beträgt 63 g (90% d. Th).

Analog dazu werden folgende Verbindungen hergestellt:
(nicht Bestandteil der beanspruchten Erfindung)

| | Edukt 1 | Edukt 2 | Produkt | Ausbeute |
|---|---|---|---|---|
| **II-2** | 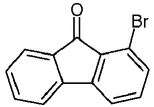 | 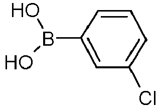 (63503-60-6) | 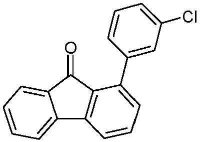 | 80% |
| **II-3** | 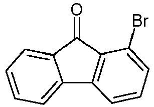 | 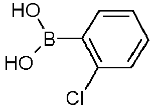 (3900-89-8) | 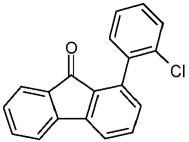 | 88% |
| **II-4** | 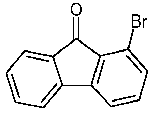 | 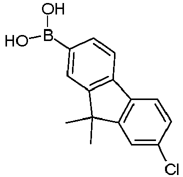 (851119-06-7) | 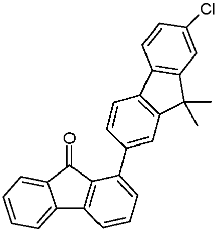 | 82% |
| **II-5** | 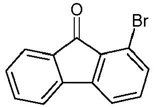 | 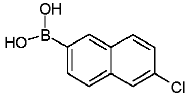 (870822-86-9) | 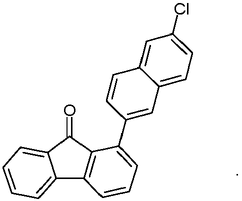 | 89% |
| **II-6** | 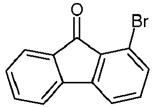 | 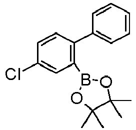 (1399362-31-2) | 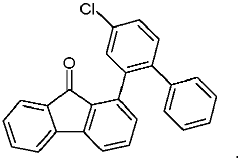 | 64% |
| **II-7** | 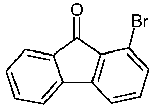 | 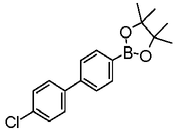 (942589-53-9) | 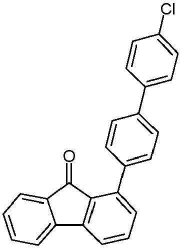 | 80% |
| **II-8** | 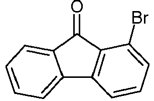 | 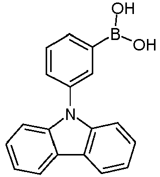 (864377-33-3) | 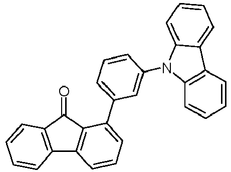 | 83% |

### Zwischenstufe III-1

30 g (120 mmol) 1-Bromo-2-diphenylether werden in einem ausgeheizten Kolben in 500 mL getrocknetem THF gelöst. Die Reaktionsmischung wird auf -78°C gekühlt. Bei dieser Temperatur werden 480 mL einer 2,5M-Lösung n-BuLi in Hexan (120 mmol) langsam zugetropft. Der Ansatz wird 1 Stunde bei -70°C nachgerührt. Anschließend werden 33 g 1-(4-Chlorphenyl)-fluorenon (114 mmol) in 100 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt.

Die einrotierte Lösung wird vorsichtig mit 300 ml Essigsäure versetzt und anschließend werden 20 ml rauchende HCl zugegeben. Der Ansatz wird auf 75°C erhitzt und 6 Stunden dort gehalten. Dabei fällt ein weißer Feststoff aus. Der Ansatz wird nun auf Raumtemperatur abgekühlt und der ausgefallene Feststoff wird abgesaugt und mit Wasser und Methanol nachgewaschen. Ausbeute: 38 g (70%).

Abschließend wird der Rückstand umkristallisiert.

Analog dazu werden folgende Verbindungen hergestellt:

| | Edukt 1 | Edukt 2 | Produkt | Ausbeute |
|---|---|---|---|---|
| **III-2** | 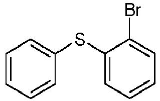 | 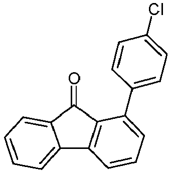 | 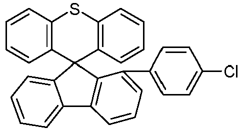 | 70% |
| **III-3** | 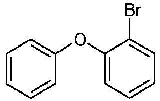 | 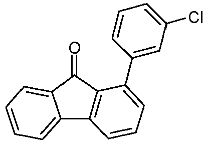 | 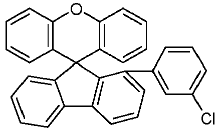 | 77% |
| **III-4** | 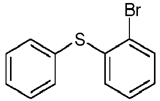 | 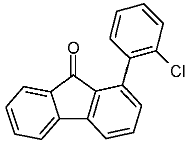 | 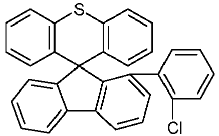 | 67% |
| **III-5** | 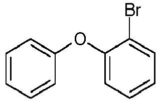 | 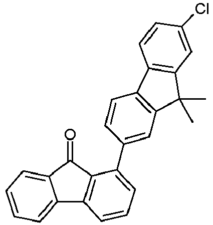 | 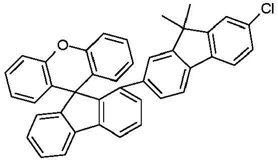 | 65% |
| **III-6** | 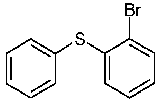 | 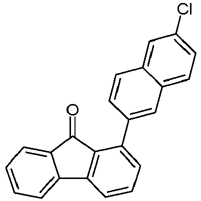 | 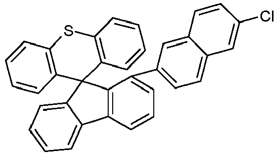 | 73% |
| **III-7** | 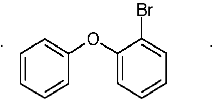 | 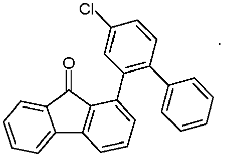 | 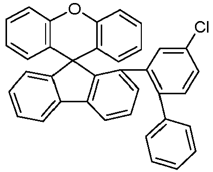 | 69% |
| **III-8** | 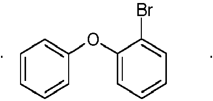 | 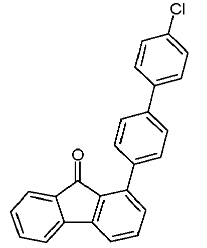 | 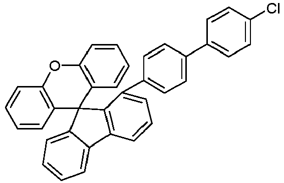 | 83% |
| **III-9** | 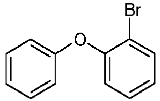 | 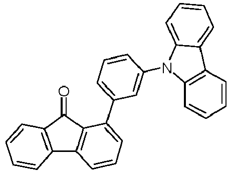 | 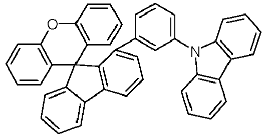 | 71% |

### Verbindung 2-1 (nicht Bestandteil der beanspruchten Erfindung)

16,3 g Biphenyl-3-yl-(9,9-dimethyl-9H-fluoren-2-yl)-amin (45,26 mmol) und 29 g des Chlor-Derivats III-1 (45,2 mmol) werden in 400 mL Toluol gelöst. Die Lösung wird entgast und mit N₂ gesättigt. Danach wird sie mit 740 mg (1,81 mmol) S-Phos und 830 mg (0,9 mmol) Pd2(dba)3 versetzt und anschließend werden 6,5 g Natrium-tert-butylat (67,7mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 27 g (78% d. Th).

Der Feststoff wird aus Heptan/Toluol umkristallisiert und abschließend im Hochvakuum sublimiert.

Analog dazu werden folgende Verbindungen hergestellt:

| | Edukt 1 | Edukt 2 | Produkt | Ausbeute |
|---|---|---|---|---|
| **2-2** | 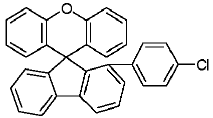 | 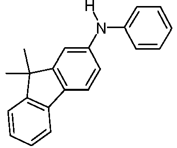 | 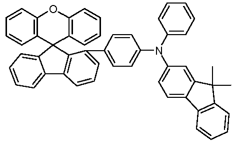 | 78% |
| **2-3** | 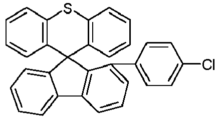 | 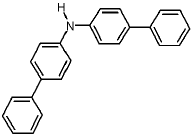 | 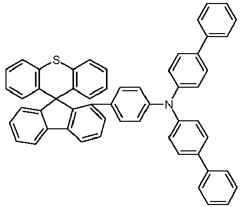 | 71% |
| **2-4** | 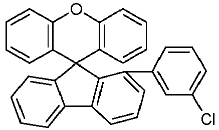 | 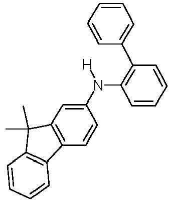 | 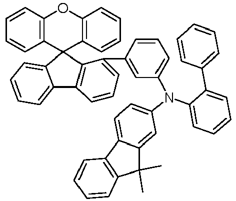 | 82% |
| **2-5** | 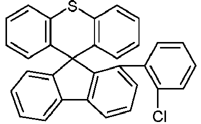 | 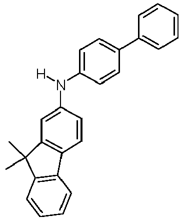 | 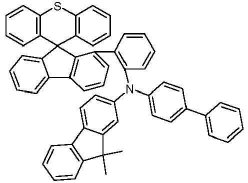 | 89% |
| **2-6** | 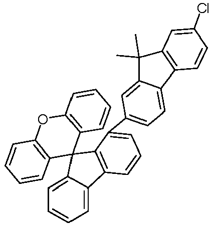 | 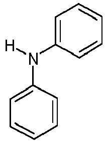 | 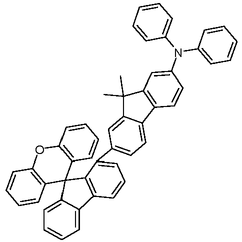 | 69% |
| **2-7** | 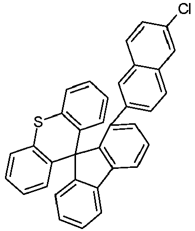 | 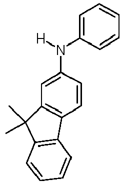 | 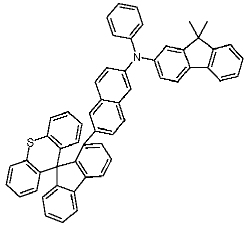 | 88% |
| **2-8** | 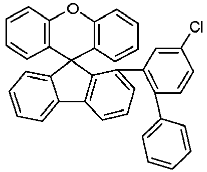 | 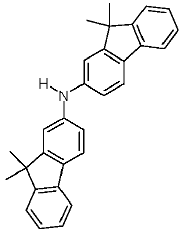 | 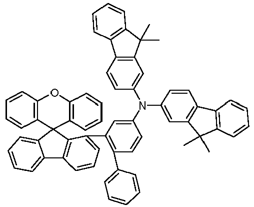 | 85% |
| **2-9** | 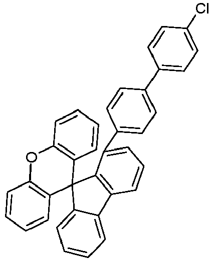 | 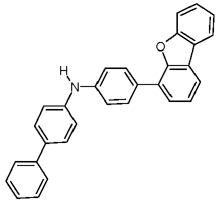 | 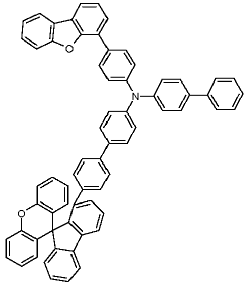 | 75% |
| **2-10** | 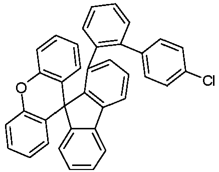 | 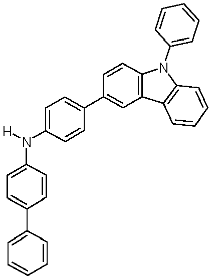 | 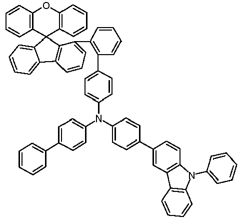 | 75% |

### B) Verwendungsbeispiele (nicht Bestandteil der beanspruchten Erfindung)

Es werden OLED-Vorrichtungen gemäß der vorliegenden Anmeldung sowie Vergleichsvorrichtungen hergestellt, um die technischen Effekte der erfindungsgemäßen OLED-Devices zu zeigen. Die OLEDs werden gemäß dem allgemeinen Verfahren, das in den Ausführungsbeispielen der Offenlegungsschrift WO 2004/058911 beschrieben ist, hergestellt, sofern unten nichts anderes angegeben ist.

Die hergestellten OLEDs haben als Substrate Glassplatten, welche mit strukturiertem ITO (Indium-Zinn-Oxid) in einer Dicke von 50 nm beschichtet sind. Die Schichten, welche auf das Substrat folgen, ihre Dicke und die Substanzen, aus denen sie bestehen, werden für jede Beispielvorrichtung in einer der folgenden Tabellen separat aufgelistet. Als Gegenelektrode wird als letzte Schicht eine Aluminiumschicht in einer Dicke von 100 nm aufgetragen.

Alle Materialien werden durch thermische Gasphasenabscheidung in einer Vakuumkammer aufgetragen. In den Beispielen besteht die Emissionsschicht immer aus wenigstens einem Matrixmaterial und einer emittierenden Verbindung als Dotand. Letztere wird dem Matrixmaterial bzw. den Matrixmaterialien durch Co-Evaporation zugefügt. Ein Ausdruck SMB:SEB (5%) bedeutet dabei, dass das Material SMB in einem Anteil von 95 Vol.-% in der Schicht vorliegt, und das Material SEB in einem Anteil von 5 Vol.-% in der Schicht vorliegt. Nicht nur die Emissionsschicht, sondern auch andere Schichten können analog aus einer Mischung von zwei oder mehr Materialien bestehen.

Die OLEDs werden nach Standardmethoden charakterisiert. Hierzu werden die Elektrolumineszenzspektren, die externe Quanteneffizienz (EQE, gemessen in %) als Funktion der Leuchtdichte, berechnet aus Strom/Spannung/Leuchdichte-Kennlinien (IUL-Kennlinien) unter Annahme Lambert'scher Emissionscharakteristik, und die Lebensdauer bestimmt. Der Ausdruck EQE @ 40 mA/cm² bedeutet dann zum Beispiel die externe Quanteneffizienz bei einer Betriebsleuchtdichte von 40 mA/cm². Die Lebensdauer wird für grün emittierende Vorrichtungen bei 20 mA/cm², und für blau emittierende Vorrichtungen bei 60 mA/cm² gemessen. Unter Annahme eines exponentiellen Abfalls bei den OLEDs werden die LT80-Werte für die Lebensdauer dann mit einem Beschleunigungsfaktor von 1.8 auf die Lebensdauer bei 1000 cd/m² hin angenähert. LT80 @ 1000 cd/m² ist dann die angenäherte Lebensdauer, bis zu der die OLED von einer initialen Leuchtdichte von 1000 cd/m² auf eine Leuchtdichte von 800 cd/m² abgefallen ist.

Die chemischen Strukturen der Materialien, die in den Beispielen eingesetzt werden, ist in Tabelle A genannt. Die Synthese der Spiroxanthen-Amine erfolgt wie im vorangehenden Synthesebeispiel-Teil, oder sie kann wie im Stand der Technik bekannt, beispielsweise wie in WO 2014/072017 offenbart, erfolgen.

| **Tabelle A** | | |
|---|---|---|
| 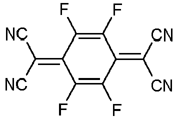 | 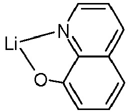 | 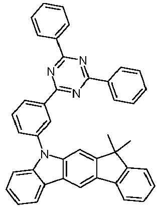 |
| F4TCNQ | LiQ | H1 |
| 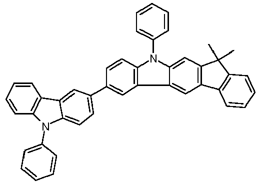 | 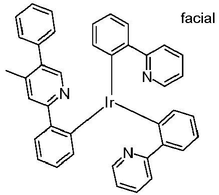 | 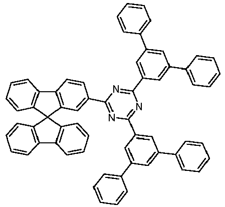 |
| H2 | TEG | ETM |
| 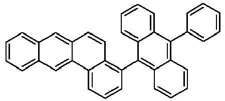 | 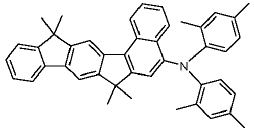 | 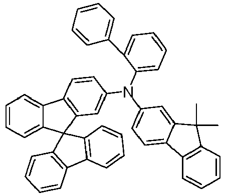 |
| SMB | SEB | HTMV1 = HIM |
| 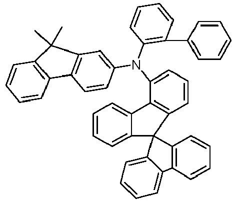 | 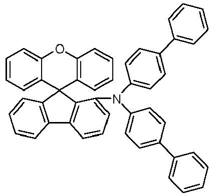 | 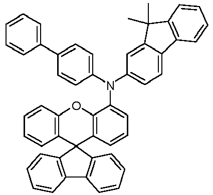 |
| HTMV2 | HTM1 | HTM2 |
| 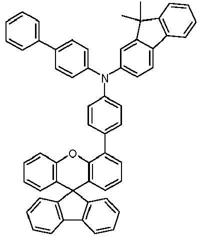 | 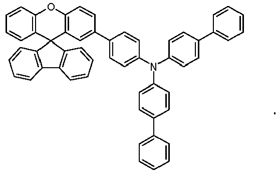 | 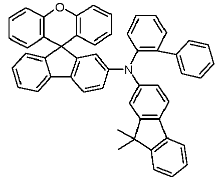 |
| HTM4 | HTM5 | HTM6 |
| 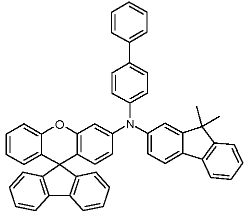 | 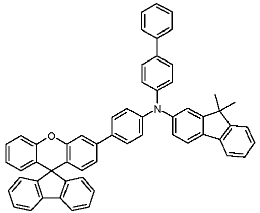 | 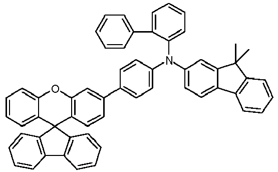 |
| HTM7 | HTM8 | HTM9 |
| 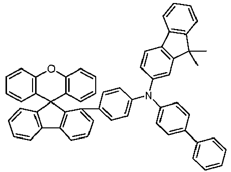 | 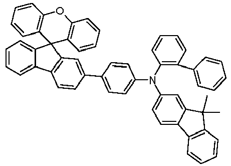 | 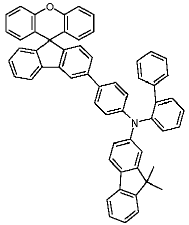 |
| HTM13 | HTM14 | HTM15 |

### 1) Verwendung von Spiroxanthen-Aminen die nicht Bestandteil der beanspruchten Erfindung sind, als HTL und HIL-

### Materialien

Es werden die folgenden OLEDs V3 (Vergleichsbeispiel) und E3, E5, E7, E9, E10, E14, E15, und E16 (nicht Bestandteil der beanspruchten Erfindung) hergestellt.

V3 als Vergleichsbeispiel enthält die Verbindung HIM (ein Spirobifluorenderivat) als HTL- und HIL-Material. Die oben genannten Verwendungsbeispiele E3, E5, E7, E9, E10, E14, E15, und E16 enthalten die Materialien HTM2, HTM4, HTM5, HTM6, HTM7, HTM8, HTM9, HTM13, HTM14, und HTM15 als HTL- und HIL-Materialien. Ansonsten ist ihr Aufbau identisch zu dem von V3 (Tabelle 1).

Für Vorrichtungen E3, E5, E7, E9, E10, E14, E15 und E16 wird ein starker Anstieg in der Lebensdauer verglichen mit dem Beispiel V3 beobachtet (Tabelle 2).

Dies zeigt die hervorragende Eignung der Spiroxanthen-Amine HTM2, HTM4, HTM6, HTM8, HTM9, HTM13 und HTM14 als HIL- und HTL-Materialien, verglichen mit dem HTL-/HIL-Material HIM gemäß dem Stand der Technik.

| **Tabelle 1: Aufbau der OLEDs** | | | | | | |
|---|---|---|---|---|---|---|
| **Bsp** | **HIL** | **HTL** | **EBL** | **EML** | **ETL** | **EIL** |
| | Dicke / nm | Dicke / nm | Dicke / nm | Dicke / nm | Dicke / nm | Dicke / nm |
| V3 | HIM:F4TCNQ(5%) | HIM | HTMV2 | SMB:SEB(5%) | ETM:LiQ(50%) | LiQ |
| | 20nm | 180nm | 10nm | 20 nm | 30 nm | 1nm |
| E3 | HTM2:F4TCNQ(5%) | HTM2 | HTMV2 | SMB:SEB(5%) | ETM:LiQ(50%) | LiQ |
| | 20nm | 180nm | 10nm | 20 nm | 30 nm | 1nm |
| E5 | HTM4:F4TCNQ(5%) | HTM4 | HTMV2 | SMB:SEB(5%) | ETM:LiQ(50%) | LiQ |
| | 20nm | 180nm | 10nm | 20 nm | 30 nm | 1nm |
| E7 | HTM6:F4TCNQ(5%) | HTM6 | HTMV2 | SMB:SEB(5%) | ETM:LiQ(50°,6) | LiQ |
| | 20nm | 180nm | 10nm | 20 nm | 30 nm | 1nm |
| E9 | HTM8:F4TCNQ(5%) | HTM8 | HTMV2 | SMB:SEB(5%) | ETM:LiQ(50°,6) | LiQ |
| | 20nm | 180nm | 10nm | 20 nm | 30 nm | 1nm |
| E10 | HTM9:F4TCNQ(5%) | HTM9 | HTMV2 | SMB:SEB(5%) | ETM:LiQ(50°,6) | LiQ |
| | 20nm | 180nm | 10nm | 20 nm | 30 nm | 1nm |
| E14 | HTM5:F4TCNQ(5%) | HTM13 | HTMV2 | SMB:SEB(5%) | ETM:LiQ(50°,6) | LiQ |
| | 20nm | 180nm | 10nm | 20 nm | 30 nm | 1nm |
| E15 | HTM6:F4TCNQ(5%) | HTM14 | HTMV2 | SMB:SEB(5%) | ETM:LiQ(50°,6) | LiQ |
| | 20nm | 180nm | 10nm | 20 nm | 30 nm | 1nm |
| E16 | HTM7:F4TCNQ(5%) | HTM15 | HTMV2 | SMB:SEB(5%) | ETM:LiQ(50°,6) | LiQ |
| | 20nm | 180nm | 10nm | 20 nm | 30 nm | 1nm |

| **Tabelle 2: Daten der OLEDs** | | |
|---|---|---|
| **Bsp.** | **U @ 10mA/cm²** | **LT80 @ 1000cd/m²** |
| | [V] | [h] |
| V3 | 3.8 | 4790 |
| E3 | 4.4 | 6800 |
| E5 | 4.3 | 4960 |
| E7 | 3.8 | 5610 |
| E9 | 4.3 | 5180 |
| E10 | 4.2 | 7390 |
| E14 | 3.9 | 5500 |
| E15 | 3.8 | 6600 |
| E16 | 4.0 | 7400 |

Ein Vergleich zwischen OLEDs, die sich lediglich durch die Tatsache, dass die Spiroxanthen-Amine in der EBL anstatt in der HTL / HIL enthalten sind, unterscheiden, ist in den folgenden Tabellen 3 und 4 gezeigt.

Tabelle 3 zeigt dabei den Aufbau der Vergleichs-OLEDs.

Tabelle 4 zeigt dabei die Ergebnisse der Direktvergleiche einander gegenübergestellt. In einer Zeile sind jeweils die miteinander zu vergleichenden Daten aufgelistet. In allen Fällen werden für den Fall, dass die Spiro-Xanthene in der HIL/HTL enthalten sind, signifikant höhere Lebensdauern erhalten (Beispiele auf der rechten Seite der Tabelle 4). Dies zeigt die Vorteile, die durch die Verwendung der Spiroxanthen-Aminverbindungen in der HIL und der HTL von OLEDs erhalten werden.

| **Tabelle 3: Aufbau der OLEDs** | | | | | | |
|---|---|---|---|---|---|---|
| **Bsp** | **HIL** | **HTL** | **EBL** | **EML** | **ETL** | **EIL** |
| | Dicke / nm | Dicke / nm | Dicke / nm | Dicke / nm | Dicke / nm | Dicke / nm |
| E17 | HIM:F4TCNQ(5°,6) 20nm | HIM 180nm | HTM2 10nm | SMB:SEB(5%) 20 nm | ETM:LiQ(50%) 30 nm | LiQ 1nm |
| E19 | HIM:F4TCNQ(5°,6) 20nm | HIM 180nm | HTM4 10nm | SMB:SEB(5%) 20 nm | ETM:LiQ(50%) 30 nm | LiQ 1nm |
| E21 | HIM:F4TCNQ(5°,6) 20nm | HIM 180nm | HTM6 20nm | SMB:SEB(5%) 20 nm | ETM:LiQ(50%) 30 nm | LiQ 1nm |
| E23 | HIM:F4TCNQ(5°,6) 20nm | HIM 180nm | HTM8 10nm | SMB:SEB(5%) 20 nm | ETM:LiQ(50%) 30 nm | LiQ 1nm |
| E24 | HIM:F4TCNQ(5°,6) 20nm | HIM 180nm | HTM9 10nm | SMB:SEB(5%) 20 nm | ETM:LiQ(50%) 30 nm | LiQ 1nm |

| **Tabelle 4: Daten der OLEDs** | | | | | |
|---|---|---|---|---|---|
| **Bsp.** | **U @ 10mA/cm²** | **LT80 @ 1000cd/m²** | **LT80 @ 1000cd/m²** | **U @ 10mA/cm²** | **Bsp.** |
| | [V] | [h] | [h] | [V] | |
| E17 | 3.9 | 3082 | 6800 | 4.4 | E3 |
| E19 | 3.8 | 2278 | 4960 | 4.3 | E5 |
| E21 | 3.9 | 2464 | 5610 | 3.8 | E7 |
| E23 | 3.7 | 3881 | 5180 | 4.3 | E9 |
| E24 | 3.7 | 4126 | 7390 | 4.2 | E10 |

### 2) Verwendung von Spiroxanthenen, die in der 1-Position mit einer

### Aminogruppe substituiert sind, und die nicht Bestandteil der beanspruchten Erfindung sind, als EBL-Materialien

Die folgenden OLEDs V1, V2, E1 und E2 werden hergestellt (Aufbau s. Tabelle 5).

V1 und V2 sind Vergleichsbeispiele, die ein 4-Spirobifluoren-Amin (HTMV2) als EBL-Material verwenden. V1 unterscheidet sich von V2 dadurch, dass ein anderes Spirobifluoren-Amin als HIL- und HTL-Material verwendet wird (HTMV1 in V1, und HTMV2 in V2).

E1 ist ein Direktvergleich zu V1. In E1 wird das Spiroxanthen-Amin HTM1 als EBL-Material anstelle des Spirobifluoren-Amins HTMV2 verwendet. E2 ist ein Direktvergleich zu V2. In E2 wird das Spiroxanthen-Amin HTM1 als EBL-Material anstelle des Spirobifluoren-Amins HTMV2 verwendet.

Sowohl für E1 als auch für E2 wird ein starker relativer Anstieg der Lebensdauer (LT80) beobachtet, verglichen mit den Beispielen V1 und V2. Parallel dazu verbessert sich die Effizienz der OLEDs (Tabelle 6).

Dies zeigt den technischen Effekt, der mit 1-Spiroxanthen-Aminen HTM1 und HTM2 erzielt wird, insbesondere bei der Verwendung als EBL-Materialien.

| **Tabelle 5: Aufbau der OLEDs** | | | | | | | |
|---|---|---|---|---|---|---|---|
| **Bsp.** | **HIL** | **HTL** | **EBL** | **EML** | **HBL** | **ETL** | **EIL** |
| | Dicke / nm | Dicke / nm | Dicke / nm | Dicke / nm | Dicke / nm | Dicke / nm | Dicke / nm |
| V1 | HTMV1: F4TCNQ(5%) 20nm | HTMV1 220nm | HTMV2 10nm | H1:H2(29%): TEG(12%) 30 nm | ETM 10 nm | ETM: LiQ(50°,6) 30 nm | LiQ 1nm |
| E1 | HTMV1: F4TCNQ(5%) 20nm | HTMV1 220nm | HTM1 10nm | H1:H2(29%): TEG(12%) 30 nm | ETM 10 nm | ETM: LiQ(50°,6) 30 nm | LiQ 1nm |
| V2 | HTMV2: F4TCNQ(5%) 20nm | HTMV2 220nm | HTMV2 10nm | H1:H2(29%): TEG(12%) 30 nm | ETM 10 nm | ETM: LiQ(50°,6) 30 nm | LiQ 1nm |
| E2 | HTMV2: F4TCNQ(5%) 20nm | HTMV2 220nm | HTM1 10nm | H1:H2(29%): TEG(12%) 30 nm | ETM 10 nm | ETM: LiQ(50°,6) 30 nm | LiQ 1nm |

| **Tabelle 6: Daten der OLEDs** | | | |
|---|---|---|---|
| **Ex.** | **U @ 2mA/cm²** | **EQE @ 2mA/cm²** | **LT80 @ 1000 cd/m²** |
| | [V] | % | [h] |
| V1 | 3.1 | 17.4 | 53400 |
| E1 | 3.3 | 18.0 | 69900 |
| V2 | 3.2 | 17.7 | 69000 |
| E2 | 3.5 | 17.9 | 76400 |

## Patentansprüche

1. Verbindung einer Formel (I) 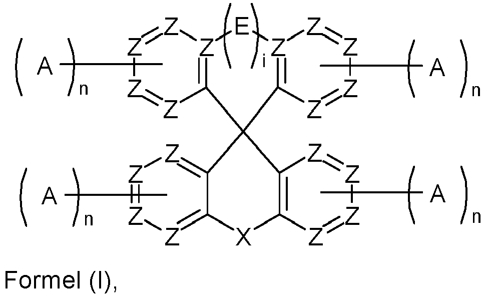 wobei gilt:
A entspricht einer Formel (A) 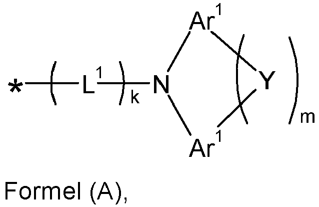 wobei gilt:
Ar¹ ist Dibenzofuranyl, das mit einem oder mehreren Resten R¹ substituiert sein kann;
Y ist gewählt aus einer Einfachbindung, BR¹, C(R¹)₂, C(R¹)₂-C(R¹)₂, Si(R¹)₂, Si(R¹)₂-Si(R¹)₂, C=O, C=NR¹, C=C(R¹)₂, C(=O)N(R¹), O, S, S=O, SO₂ und NR¹;
k ist gleich 0;
m ist gleich 0 oder 1; wobei die Gruppe A über die mit * markierte Bindung an den Rest der Verbindung der Formel (I) gebunden ist;
E ist eine Einfachbindung;
X ist O oder S;
Z ist bei jedem Auftreten gleich oder verschieden CR² oder N oder C, wobei eine Gruppe Z genau dann gleich C ist, wenn eine Gruppe A oder E an die betreffende Gruppe Z gebunden ist;
R¹ ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können;
R² ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkyl-, Alkoxy-, Alkenyl-und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C=C-, Si(R³)₂, C=O, C=NR³, -C(=O)O-, -C(=O)NR³-, NR³, P(=O)(R³), -O-, -S-, SO oder SO₂ ersetzt sein können;
R³ ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R³ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, CN, Alkyl- oder Alkoxygruppen mit 1 bis 20 C-Atomen, Alkenyl- oder Alkinylgruppen mit 2 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkyl-, Alkoxy-, Alkenyl- und Alkinylgruppen, aromatischen Ringsysteme und heteroaromatischen Ringsysteme mit F oder CN substituiert sein können;
i ist gleich 1;
n ist bei jedem Auftreten gleich oder verschieden 0 oder 1, wobei die Summe aller indices n gleich 1, 2, 3 oder 4 ist.

2. Verbindung nach Anspruch 1, **dadurch gekennzeichnet, dass** X gleich O ist.

3. Verbindung nach Anspruch 1 oder 2, **dadurch gekennzeichnet, dass** die Summe der indices n gleich 1 ist.

4. Verbindung nach einem oder mehreren der Ansprüche 1 bis 3, **dadurch gekennzeichnet, dass** R¹ bei jedem Auftreten gleich oder verschieden gewählt ist aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R³ substituiert sein können.

5. Verbindung nach einem oder mehreren der Ansprüche 1 bis 4, **dadurch gekennzeichnet, dass** R² bei jedem Auftreten gleich oder verschieden gewählt ist aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R³ substituiert sein können.

6. Verbindung nach einem oder mehreren der Ansprüche 1 bis 5, **dadurch gekennzeichnet, dass** R³ bei jedem Auftreten gleich oder verschieden gewählt ist aus H, F, CN, geradkettigen Alkylgruppen mit 1 bis 20 C-Atomen, verzweigten oder cyclischen Alkylgruppen mit 3 bis 20 C-Atomen, aromatischen Ringsystemen mit 6 bis 40 aromatischen Ringatomen, und heteroaromatischen Ringsystemen mit 5 bis 40 aromatischen Ringatomen; wobei die genannten Alkylgruppen, die genannten aromatischen Ringsysteme und die genannten heteroaromatischen Ringsysteme jeweils mit einem oder mehreren Resten R⁴ substituiert sein können.

7. Verbindung nach einem oder mehreren der Ansprüche 1 bis 6, **dadurch gekennzeichnet, dass** die Verbindung der Formel (I) einer der folgenden Formeln entspricht 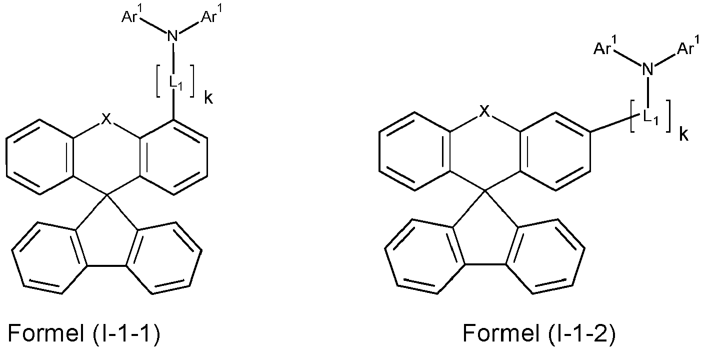 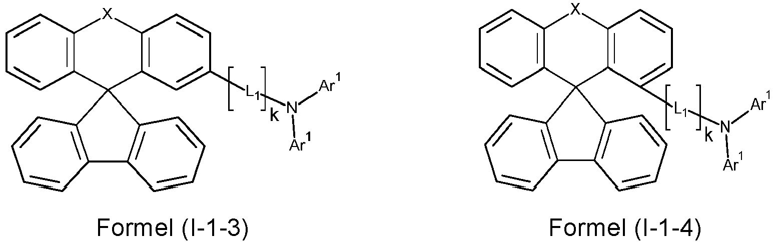 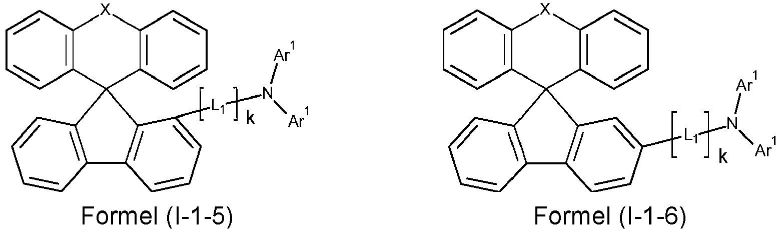 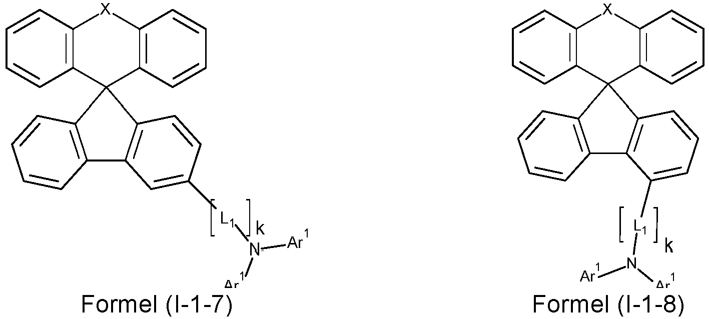 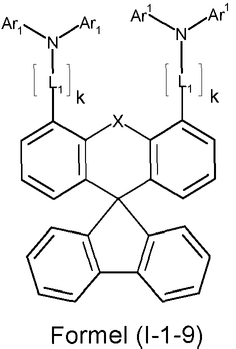 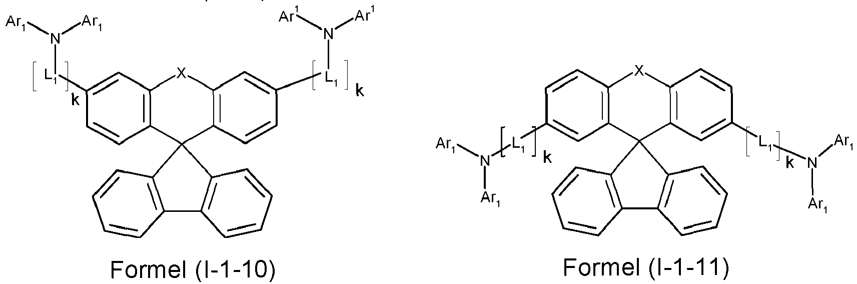 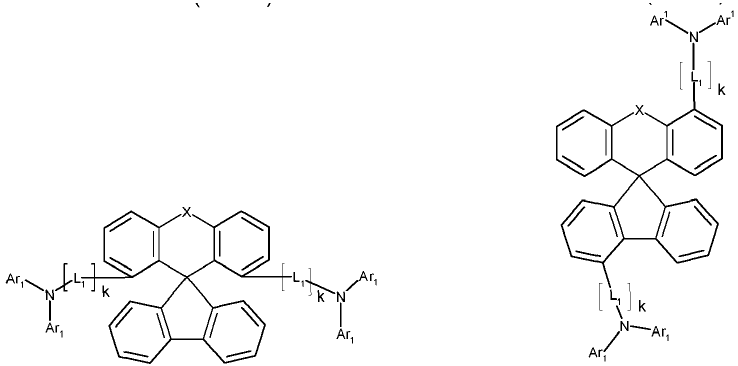 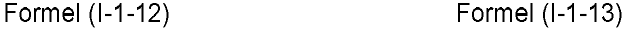 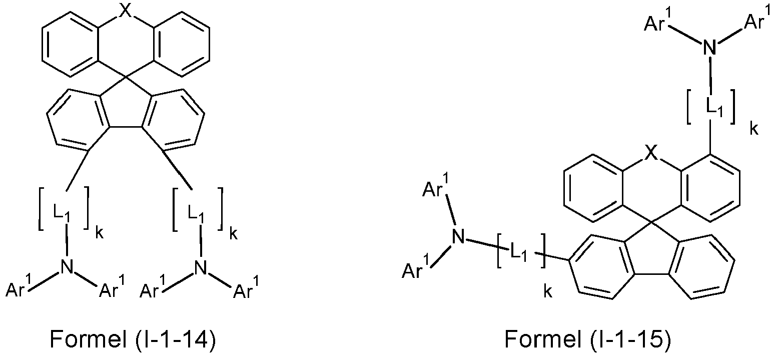 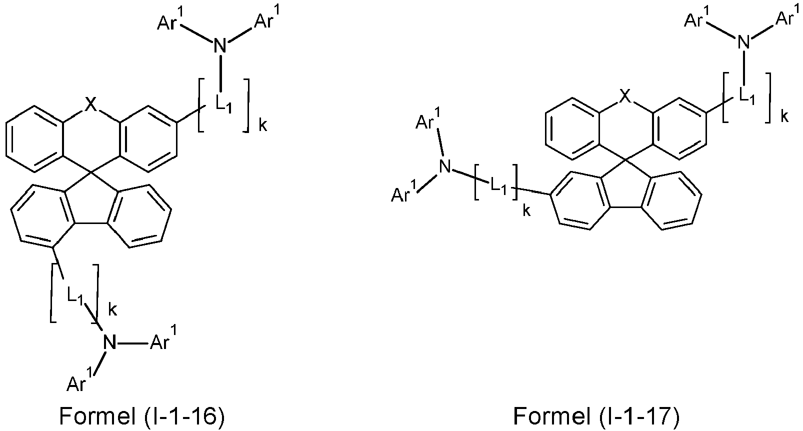 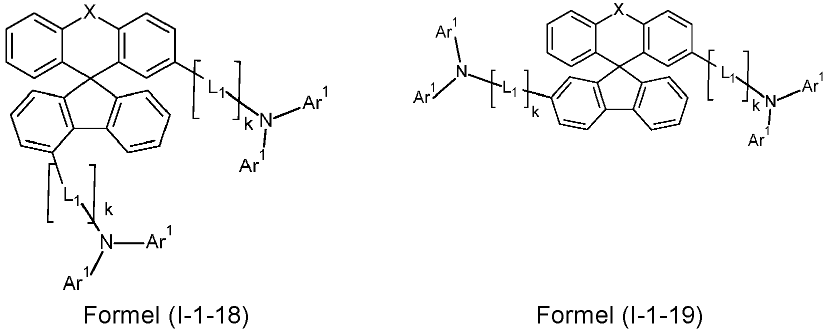 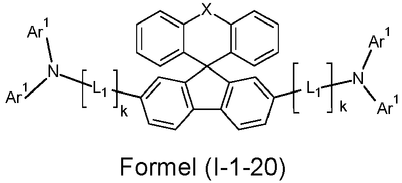 wobei X, L¹, Ar¹ und k definiert sind wie in einem oder mehreren der Ansprüche 1 bis 6, und wobei die Verbindungen an den unsubstituiert gezeichneten Positionen an den Benzolringen jeweils mit Resten R², definiert wie in Anspruch 1 oder 5, substituiert sein können, und bevorzugt in diesen Positionen unsubstituiert sind.

8. Verbindung nach Anspruch 7, **dadurch gekennzeichnet, dass** X gleich O ist, und sie an den unsubstituiert gezeichneten Positionen an den Benzolringen jeweils unsubstituiert ist.

9. Verbindung nach einem oder mehreren der Ansprüche 1 bis 8, **dadurch gekennzeichnet, dass** m gleich 0 ist.

10. Elektronische Vorrichtung, gewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs), **dadurch gekennzeichnet, dass** sie eine Verbindung nach einem oder mehreren der Ansprüche 1 bis 9 enthält.

11. Elektronische Vorrichtung gemäß Anspruch 10, **dadurch gekennzeichnet, dass** sie eine organische Elektrolumineszenzvorrichtung (OLED) ist.

12. Verwendung einer Verbindung gemäß einem oder mehreren der Ansprüche 1 bis 9 in einer elektronischen Vorrichtung, bevorzugt einer organischen Elektrolumineszenzvorrichtung (OLED).

## Claims

1. Compound of a formula (I) 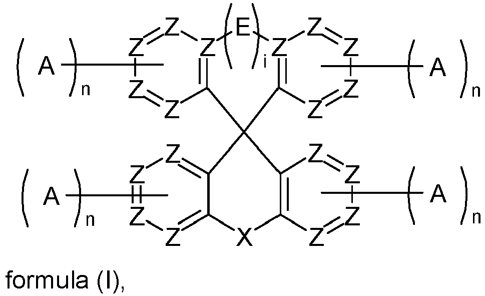 where the following applies:
A corresponds to a formula (A) 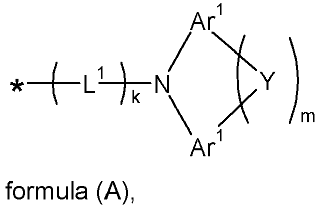 where the following applies:
Ar¹ is dibenzofuranyl, which may be substituted by one or more radicals R¹;
Y is selected from a single bond, BR¹, C(R¹)₂, C(R¹)₂-C(R¹)₂, Si(R¹)₂, Si(R¹)₂-Si(R¹)₂, C=O, C=NR¹, C=C(R¹)₂, C(=O)N(R'), O, S, S=O, SO₂ and NR¹;
k is equal to 0;
m is equal to 0 or 1; where the group A is bonded to the remainder of the compound of the formula (I) via the bond marked with *;
E is a single bond;
X is O or S;
Z is on each occurrence, identically or differently, CR² or N or C, where a group Z is equal to C precisely if a group A or E is bonded to the group Z in question;
R¹ is selected on each occurrence, identically or differently, from H, D, F, C(=O)R³, CN, Si(R³)₃, N(R³)₂, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ may be linked to one another and may form a ring; where the said alkyl, alkoxy, alkenyl and alkynyl groups and the said aromatic ring systems and heteroaromatic ring systems may in each case be substituted by one or more radicals R³; and where one or more CH₂ groups in the said alkyl, alkoxy, alkenyl and alkynyl groups may be replaced by -R³C=CR³-, -C=C-, Si(R³)₂, C=O, C=NR³, -C(=O)O-, -C(=O)NR³-, NR³, P(=O)(R³), -O-, -S-, SO or SO₂;
R² is selected on each occurrence, identically or differently, from H, D, F, C(=O)R³, CN, Si(R³)₃, N(R³)₂, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where the said alkyl, alkoxy, alkenyl and alkynyl groups and the said aromatic ring systems and heteroaromatic ring systems may in each case be substituted by one or more radicals R³; and where one or more CH₂ groups in the said alkyl, alkoxy, alkenyl and alkynyl groups may be replaced by -R³C=CR³-, -C=C-, Si(R³)₂, C=O, C=NR³, -C(=O)O-, -C(=O)NR³-, NR³, P(=O)(R³), -O-, -S-, SO or SO₂;
R³ is selected on each occurrence, identically or differently, from H, D, F, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R³ may be linked to one another and may form a ring; where the said alkyl, alkoxy, alkenyl and alkynyl groups and the said aromatic ring systems and heteroaromatic ring systems may in each case be substituted by one or more radicals R⁴; and where one or more CH₂ groups in the said alkyl, alkoxy, alkenyl and alkynyl groups may be replaced by -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO or SO₂;
R⁴ is selected on each occurrence, identically or differently, from H, D, F, CN, alkyl or alkoxy groups having 1 to 20 C atoms, alkenyl or alkynyl groups having 2 to 20 C atoms, aromatic ring systems having 6 to 40 aromatic ring atoms and heteroaromatic ring systems having 5 to 40 aromatic ring atoms; where two or more radicals R⁴ may be linked to one another and may form a ring; and where the said alkyl, alkoxy, alkenyl and alkynyl groups, aromatic ring systems and heteroaromatic ring systems may be substituted by F or CN;
i is equal to 1;
n is on each occurrence, identically or differently, 0 or 1, where the sum of all indices n is equal to 1, 2, 3 or 4.

2. Compound according to Claim 1, **characterised in that** X is equal to O.

3. Compound according to Claim 1 or 2, **characterised in that** the sum of the indices n is equal to 1.

4. Compound according to one or more of Claims 1 to 3, **characterised in that** R¹ is selected on each occurrence, identically or differently, from H, F, CN, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where the said alkyl groups, the said aromatic ring systems and the said heteroaromatic ring systems may in each case be substituted by one or more radicals R³.

5. Compound according to one or more of Claims 1 to 4, **characterised in that** R² is selected on each occurrence, identically or differently, from H, F, CN, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where the said alkyl groups, the said aromatic ring systems and the said heteroaromatic ring systems may in each case be substituted by one or more radicals R³.

6. Compound according to one or more of Claims 1 to 5, **characterised in that** R³ is selected on each occurrence, identically or differently, from H, F, CN, straight-chain alkyl groups having 1 to 20 C atoms, branched or cyclic alkyl groups having 3 to 20 C atoms, aromatic ring systems having 6 to 40 aromatic ring atoms, and heteroaromatic ring systems having 5 to 40 aromatic ring atoms; where the said alkyl groups, the said aromatic ring systems and the said heteroaromatic ring systems may in each case be substituted by one or more radicals R⁴.

7. Compound according to one or more of Claims 1 to 6, **characterised in that** the compound of the formula (I) corresponds to one of the following formulae 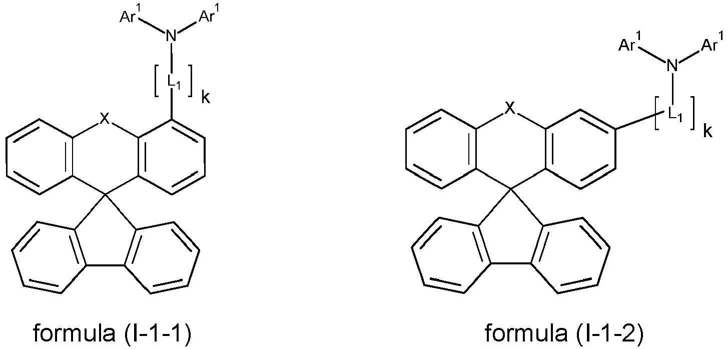 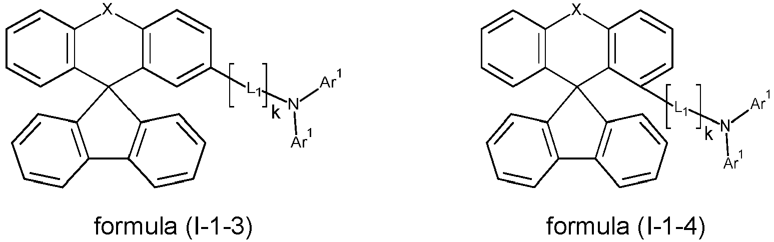 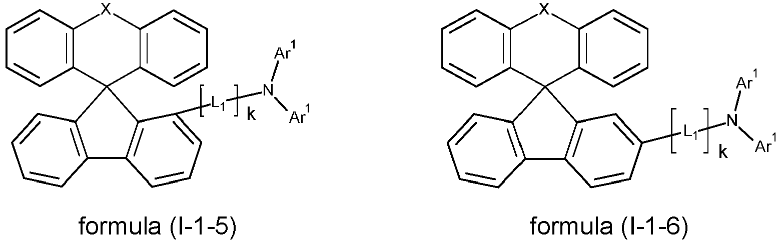 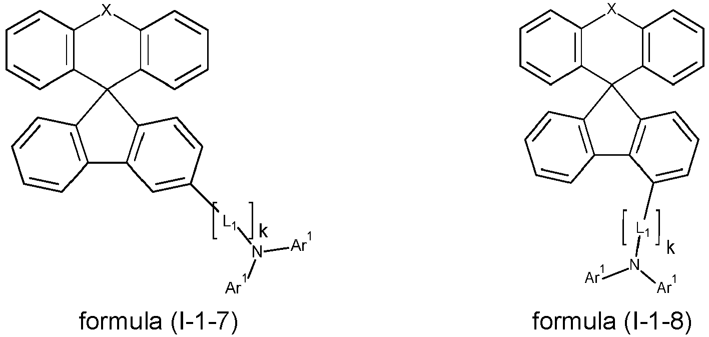 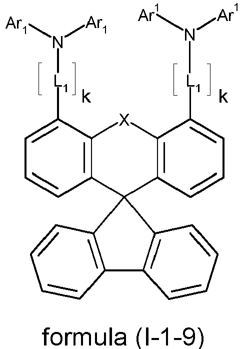 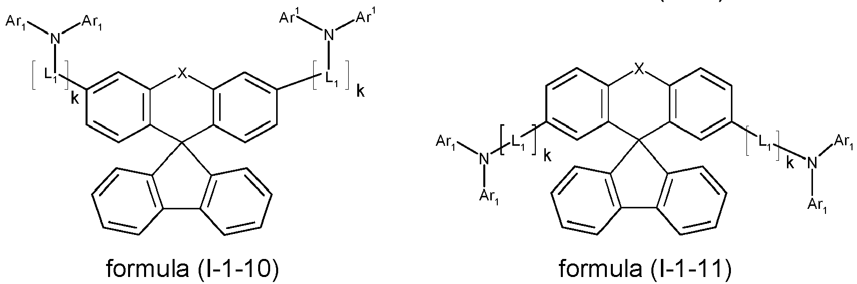 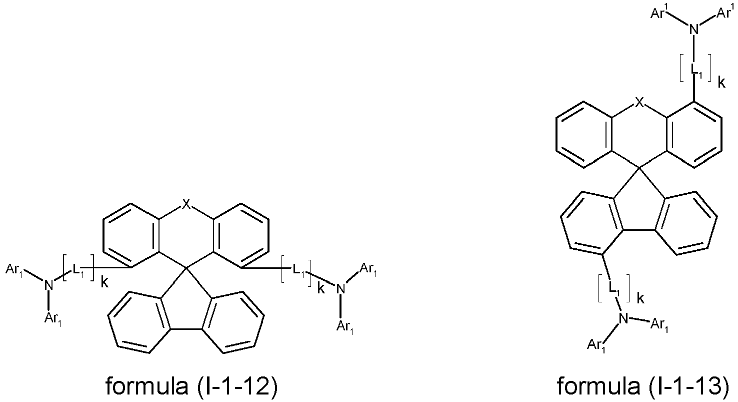 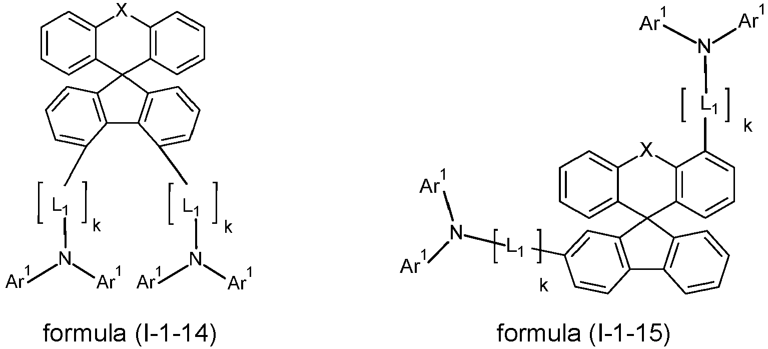 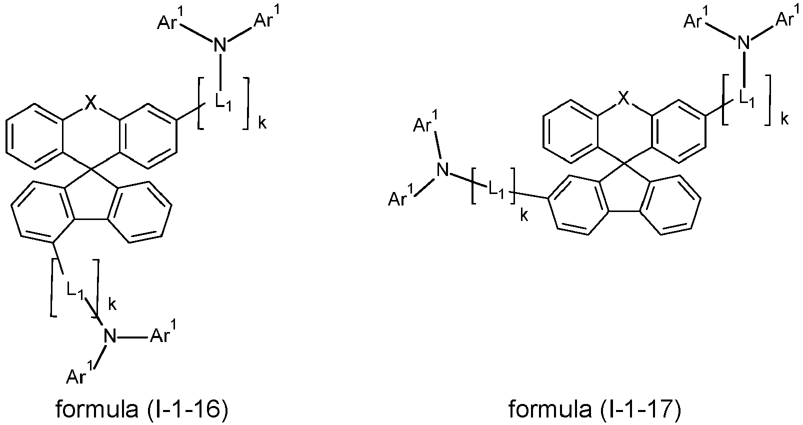 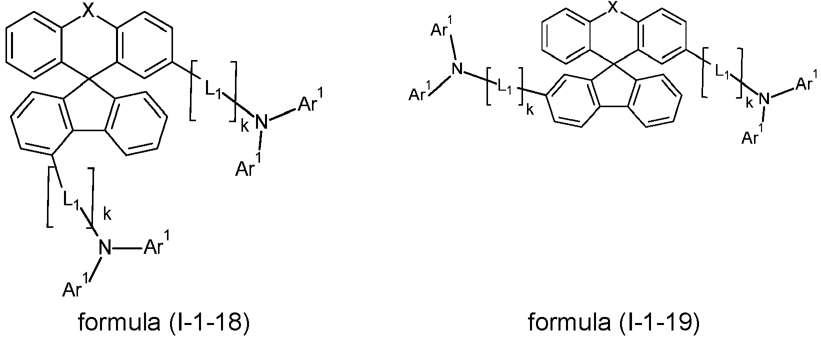 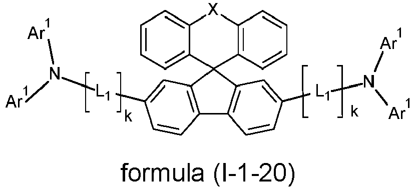 where X, L', Ar¹ and k are defined as in one or more of Claims 1 to 6, and where the compounds may be substituted by radicals R², defined as in Claim 1 or 5, at each of the positions that are drawn as unsubstituted on the benzene rings, and are preferably unsubstituted in these positions.

8. Compound according to Claim 7, **characterised in that** X is equal to O, and it is unsubstituted at each of the positions that are drawn as unsubstituted on the benzene rings.

9. Compound according to one or more of Claims 1 to 8, **characterised in that** m is equal to 0.

10. Electronic device selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs), **characterised in that** it contains a compound according to one or more of Claims 1 to 9.

11. Electronic device according to Claim 10, **characterised in that** it is an organic electroluminescent device (OLED).

12. Use of a compound according to one or more of Claims 1 to 9 in an electronic device, preferably an organic electroluminescent device (OLED).

## Revendications

1. Composé de formule (I) 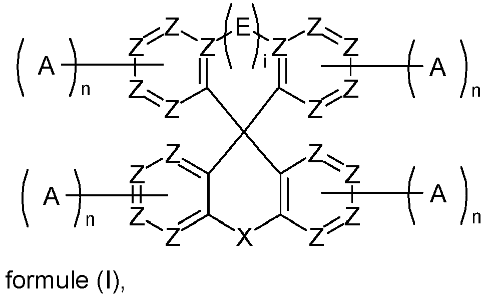 dans laquelle ce qui suit s'applique :
A correspond à une formule (A) 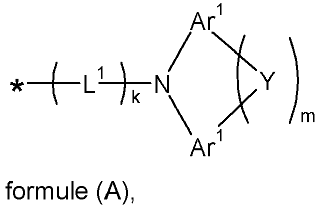 dans laquelle ce qui suit s'applique :
Ar¹ est dibenzofuranyle, qui peut être substitué par un ou plusieurs radicaux R¹ ;
Y est choisi parmi une liaison simple, BR¹, C(R¹)₂, C(R¹)₂-C(R¹)₂, Si(R¹)₂, Si(R¹)₂-Si(R¹)₂, C=O, C=NR¹, C=C(R¹)₂, C(=O)N(R'), O, S, S=O, SO₂ et NR' ;
k est égal à 0 ;
m est égal à 0 ou 1 ; où le groupement A est lié au reste du composé de formule (I) via la liaison marquée par * ;
E est une liaison simple ;
X est O ou S ;
Z est à chaque occurrence, de manière identique ou différente, CR² ou N ou C, où un groupement Z est égal à C précisément si un groupement A ou E est lié au groupement Z en question ;
R¹ est choisi à chaque occurrence, de manière identique ou différente, parmi H, D, F, C(=O)R³, CN, Si(R³)₃, N(R³)₂, P(=O)(R³)₂, OR³, S(=O)R³, S(=O)₂R³, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R¹ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R³ ; et où un ou plusieurs groupements CH₂ dans lesdits groupements alkyle, alcoxy, alcényle et alcynyle peuvent être remplacés par -R³C=CR³-, -C=C-, Si(R³)₂, C=O, C=NR³, -C(=O)O-, -C(=O)NR³-, NR³, P(=O)(R³), -O-, -S-, SO ou SO₂ ;
R² est choisi à chaque occurrence, de manière identique ou différente, parmi H, D, F, C(=O)R³, CN, Si(R³)₃, N(R³)₂, P(=O)(R³)₂, OR³, S(=O)R³, S(=O)₂R³, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, alcoxy, alcényle et alcynyle et lesdits noyaux aromatiques et noyaux hétéroaromatiques peuvent dans chaque cas être substitués par un ou plusieurs radicaux R³ ; et où un ou plusieurs groupements CH₂ dans lesdits groupements alkyle, alcoxy, alcényle et alcynyle peuvent être remplacés par -R³C=CR³-, -C=C-, Si(R³)₂, C=O, C=NR³, -C(=O)O-, -C(=O)NR³-, NR³, P(=O)(R³), -O-, -S-, SO ou SO₂ ;
R³ est choisi à chaque occurrence, de manière identique ou différente, parmi H, D, F, C(=O)R⁴, CN, Si(R⁴)₃, N(R⁴)₂, P(=O)(R⁴)₂, OR⁴, S(=O)R⁴, S(=O)₂R⁴, des groupements alkyle ou alcoxy à chaîne linéaire ayant de 1 à 20 atomes de C, des groupements alkyle ou alcoxy ramifiés ou cycliques ayant de 3 à 20 atomes de C, des groupements alcényle ou alcynyle ayant de 2 à 20 atomes de C, des noyaux aromatiques ayant de 6 à 40 atomes de cycle aromatique, et des noyaux hétéroaromatiques ayant de 5 à 40 atomes de cycle aromatique ; où deux, ou plus, radicaux R³ peuvent être liés les uns aux autres et peuvent former un cycle ; où lesdits groupements alkyle, alcoxy, alcényle et alcynyle et lesdits noyaux aromatiques et noyaux hétéroaromatiques peuvent dans chaque cas être substitués par un ou plusieurs radicaux R⁴ ; et où un ou plusieurs groupements CH₂ dans lesdits groupements alkyle, alcoxy, alcényle et alcynyle peuvent être remplacés par -R⁴C=CR⁴-, -C=C-, Si(R⁴)₂, C=O, C=NR⁴, -C(=O)O-, -C(=O)NR⁴-, NR⁴, P(=O)(R⁴), -O-, -S-, SO ou SO₂ ;
R⁴ est choisi à chaque occurrence, de manière identique ou différente, parmi H, D, F, CN, des groupements alkyle ou alcoxy ayant de 1 à 20 atomes de C, des groupements alcényle ou alcynyle ayant de 2 à 20 atomes de C, des noyaux aromatiques ayant de 6 à 40 atomes de cycle aromatique et des noyaux hétéroaromatiques ayant de 5 à 40 atomes de cycle aromatique ; où deux, ou plus, radicaux R⁴ peuvent être liés les uns aux autres et peuvent former un cycle ; et où lesdits groupements alkyle, alcoxy, alcényle et alcynyle, noyaux aromatiques et noyaux hétéroaromatiques peuvent être substitués par F ou CN ;
i est égal à 1 ;
n vaut à chaque occurrence, de manière identique ou différente, 0 ou 1, où la somme de tous les indices n est égale à 1, 2, 3 ou 4.

2. Composé selon la revendication 1, **caractérisé en ce que** X est égal à O.

3. Composé selon la revendication 1 ou 2, **caractérisé en ce que** la somme des indices n est égale à 1.

4. Composé selon l'une ou plusieurs parmi les revendications 1 à 3, **caractérisé en ce que** R¹ est choisi à chaque occurrence, de manière identique ou différente, parmi H, F, CN, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, lesdits noyaux aromatiques et lesdits noyaux hétéroaromatiques peuvent dans chaque cas être substitués par un ou plusieurs radicaux R³.

5. Composé selon l'une ou plusieurs parmi les revendications 1 à 4, **caractérisé en ce que** R² est choisi à chaque occurrence, de manière identique ou différente, parmi H, F, CN, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, lesdits noyaux aromatiques et lesdits noyaux hétéroaromatiques peuvent dans chaque cas être substitués par un ou plusieurs radicaux R³.

6. Composé selon l'une ou plusieurs parmi les revendications 1 à 5, **caractérisé en ce que** R³ est choisi à chaque occurrence, de manière identique ou différente, parmi H, F, CN, des groupements alkyle à chaîne linéaire ayant de 1 à 20 atomes de C, des groupements alkyle ramifiés ou cycliques ayant de 3 à 20 atomes de C, des noyaux aromatiques ayant de 6 à 40 atomes de cycle aromatique, et des noyaux hétéroaromatiques ayant de 5 à 40 atomes de cycle aromatique ; où lesdits groupements alkyle, lesdits noyaux aromatiques et lesdits noyaux hétéroaromatiques peuvent dans chaque cas être substitués par un ou plusieurs radicaux R⁴.

7. Composé selon l'une ou plusieurs parmi les revendications 1 à 6, **caractérisé en ce que** le composé de formule (I) correspond à l'une des formules suivantes 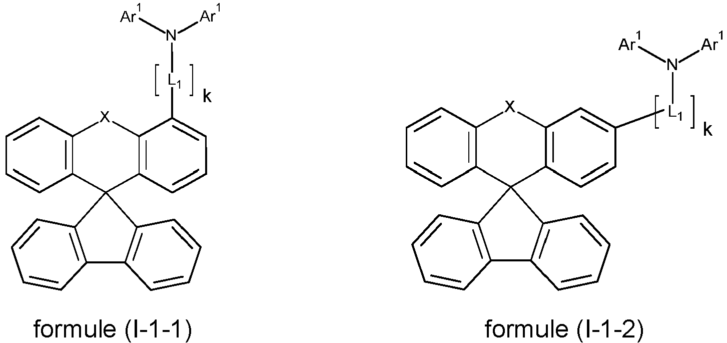 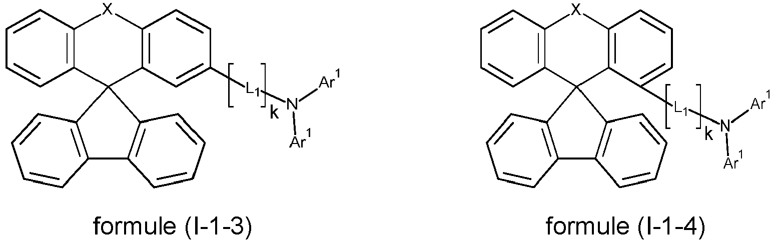 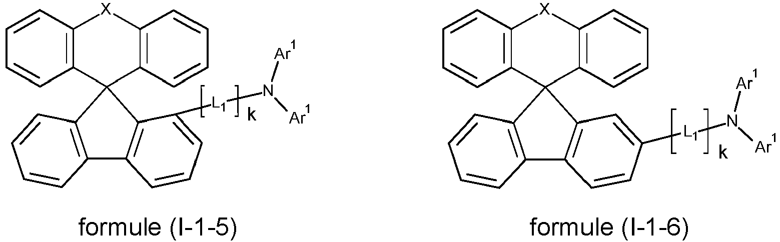 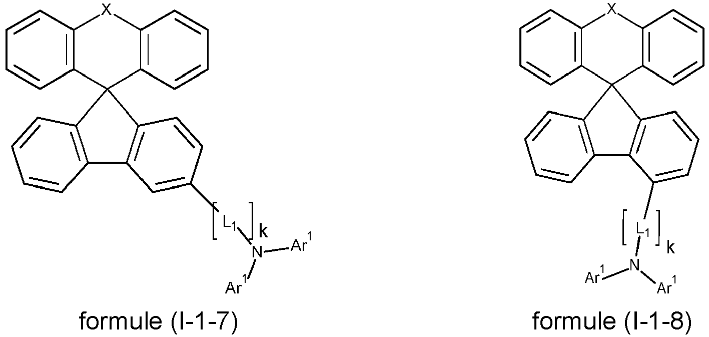 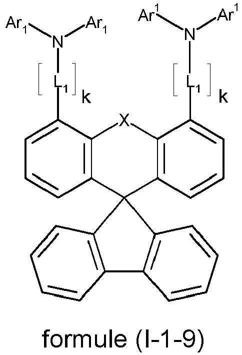 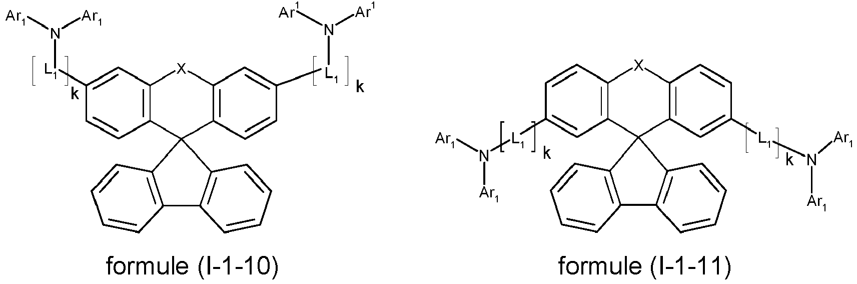 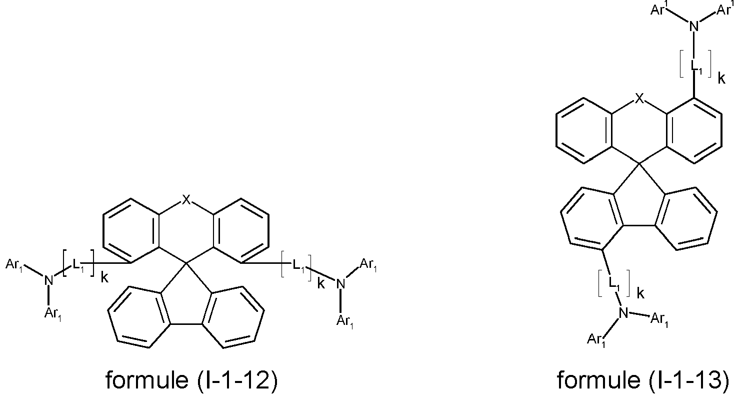 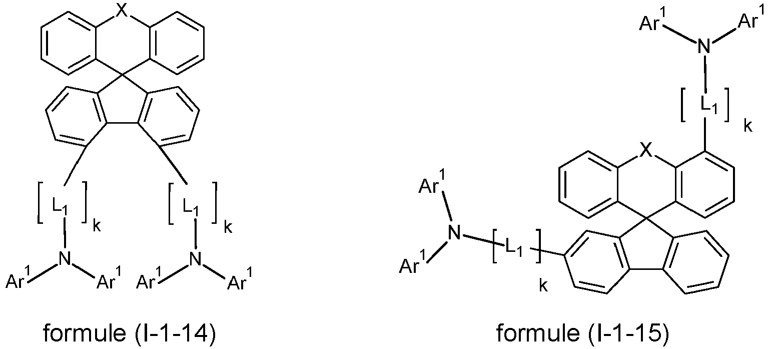 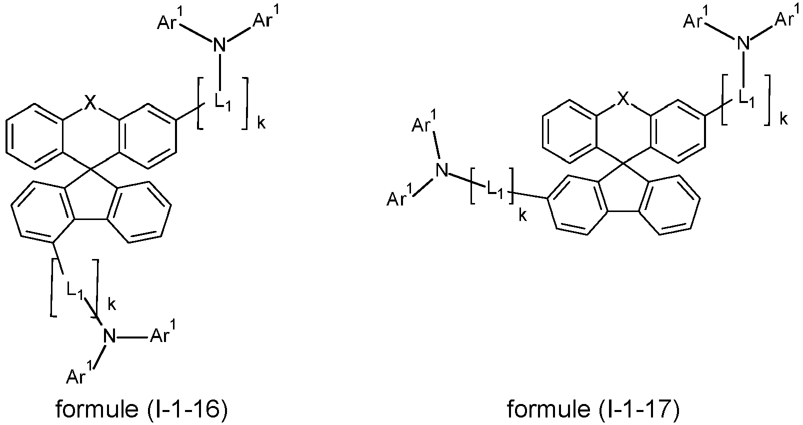 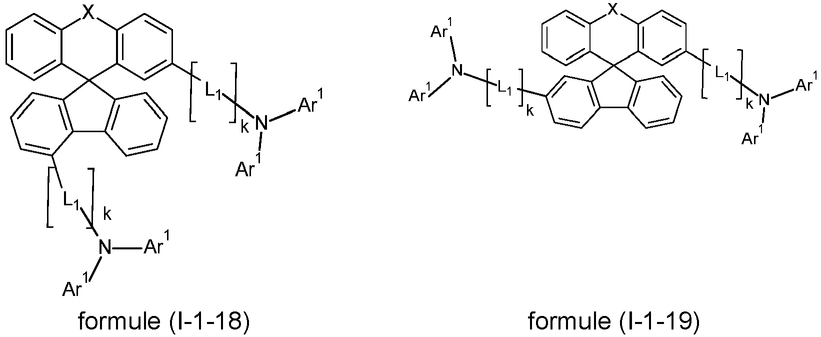 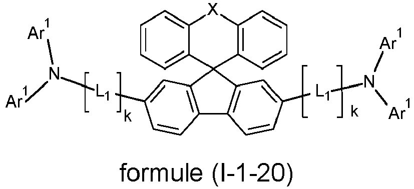 dans lesquelles X, L', Ar¹ et k sont tels que définis selon l'une ou plusieurs parmi les revendications 1 à 6, et où les composés peuvent être substitués par des radicaux R², définis selon la revendication 1 ou 5, au niveau de chacune des positions qui sont dessinées comme étant non substituées sur les cycles benzéniques, et sont préférablement non substitués dans ces positions.

8. Composé selon la revendication 7, **caractérisé en ce que** X est égal à O, et est non substitué au niveau de chacune des positions qui sont dessinées comme étant non substituées sur les cycles benzéniques.

9. Composé selon l'une ou plusieurs parmi les revendications 1 à 8, **caractérisé en ce que** m est égal à 0.

10. Dispositif électronique choisi dans le groupe constitué par les circuits intégrés organiques (OIC), les transistors organiques à effet de champ (OFET), les transistors organiques à couche mince (OTFT), les transistors organiques émetteurs de lumière (OLET), les cellules solaires organiques (OSC), les détecteurs optiques organiques, les photorécepteurs organiques, les dispositifs organiques à extinction de champ (OFQD), les cellules électrochimiques organiques émettrices de lumière (OLEC), les diodes laser organiques (O-lasers) et les dispositifs électroluminescents organiques (OLED), **caractérisé en ce qu'**il contient un composé selon l'une ou plusieurs parmi les revendications 1 à 9.

11. Dispositif électronique selon la revendication 10, **caractérisé en ce qu'**il s'agit d'un dispositif électroluminescent organique (OLED).

12. Utilisation d'un composé selon l'une ou plusieurs parmi les revendications 1 à 9, dans un dispositif électronique, préférablement un dispositif électroluminescent organique (OLED).
